# EUROPEAN PATENT SPECIFICATION

(11) **EP 4 192 788 B1**
(45) Date of publication and mention of the grant of the patent: **23.07.2025**
(21) Application number: 21759269.0
(22) Date of filing: 05.08.2021
(51) Int. Cl.: C01B 33/32, A61K 6/76, A61K 33/00, A61L 24/02, A61L 27/02, A61L 27/56, A61L 27/58, A61P 43/00, A61L 24/00

(54) **LITHIATED SILICON**
LITHIUMHALTIGES SILIZIUM
SILICIUM LITHIÉ

(30) Priority: 07.08.2020 GB 202012302
(43) Date of publication of application: 14.06.2023
(73) Proprietor: King's College London, London WC2R 2LS (GB)
(72) Inventor: CHIAPPINI, Ciro, London SE1 9RT (GB); KAASALAINEN, Martti, London SE1 9RT (GB); SHARPE, Paul, London SE1 9RT (GB)
(74) Representative: Greaves Brewster LLP
(86) International application number: PCT/EP2021/071961
(87) International publication number: WO 2022/029277

(56) References cited:
- EP-A1- 3 507 845
- EP-A1- 3 678 229
- US-A1- 2013 034 714
- DATABASE WPI Week 2020, Derwent World Patents Index; AN 2020-035268, XP002804771
- SFAMPA IOANNA K ET AL: "Study of dental zirconia reinforced lithium silicate for its use as a personal accidental dosimeter", APPLIED RADIATION AND ISOTOPES, ELSEVIER, OXFORD, GB, vol. 157, 11 December 2019 (2019-12-11), XP086045555, ISSN: 0969-8043, [retrieved on 20191211], DOI: 10.1016/J.APRADISO.2019.109024

## Description

### TECHNICAL FIELD

This invention relates to lithiated porous silicon particles comprising lithium, silicon and oxygen. The invention also relates to a method of preparing lithiated porous silicon particles and use of lithiated porous silicon particles, particularly in repairing or regenerating mineralised tissue.

### BACKGROUND

Controlled stimulation of cell signalling pathways is relevant for bone regeneration. Recently, significant progress has been made in the local delivery of recombinant bone morphogenetic protein 2 (rBMP-2), including the FDA approved Infused Bone Graft (Medtronic) in USA and EU approval (Wyeth) in Europe. This product highlights how, by stimulating osteogenic master regulatory pathways, it is possible to generate large amounts of mineralised tissue. Yet this approach requires supra physiological BMP-2 amounts, which carries risk of severe side effects, such as generalized hematomas in soft tissue, exaggerated inflammatory response in proximal humeral fractures, unicameral bone cyst, and infections in open tibial fractures. Furthermore, in 2013 the FDA denied a premarketing approval of high dose rBMP-2 product following the high incidence of cancer cases in a group of patients. These, together with the fact that recombinant proteins are complex and expensive to fabricate and have limited shelf life, limit their applications. In addition, in order to be able to stimulate nonexposed resident progenitor cells such as those in dental pulp during reparative dentine formation, large biological drug molecules can have only a minor role because they are less effectively diffused through the remaining dentine.

The Wnt/β-catenin pathway is a master regulator of osteogenesis, and glycogen synthase kinase (GSK-3) inhibition is an established strategy to stimulate the pathway to induce mineral tissue formation. Inhibition of GSK-3 leads to nuclear accumulation of β-catenin and activation of the TCF/Lef transcription factors, which modulate the expression of Runx2, Sox9 and Axin2 genes. Temporally defined Runx2 and Sox9 expression patterns direct mesenchymal stem cells (MSCs) towards osteoblast specification. GSK-3 is an important drug target also in diabetes and neurodegenerative diseases. It has been shown that small molecule GSK-3 inhibitors can stimulate Wnt/β-catenin pathway and can regenerate dental tissue. Lithium is a dual-inhibitor of GSK-3 activity which is highly effective at activating wnt/ β-catenin. This regeneration significantly outperforms the natural regenerative capacity and is obtained using cheaper and safer inhibitors compared to recombinant proteins.

Oral intake of lithium has been found to stimulate bone formation in mice and to decrease the human bone fracture risk.

EP3678229 discloses a pre-lithiated silicon-oxygen anode active material. US2013/034714 discloses a method of making porous silicon using a chemical etchant comprising metal ions. Sfampa et al. (Applied Radiation and Isotopes, Elsevevier, Oxford, GB, Vol. 157, 11 December 2019) discloses a study of dental zirconia reinforced lithium silicate for use as a personal accidental dosimeter.

Bioactive glass is one of the devices used to control the delivery of lithium for regenerative purposes. Bioactive glasses (also referred to as bioglasses) are glass-ceramic materials comprising different compounds. The first and the most studied bioactive glass is 4555 Bioglass^{®} which consists of (wt. %) 45 SiO₂, 24.5 CaO, 24.5 Na₂O, 6 P₂O₅. Bioactive glasses are known to leech out their constituents when in biological environments, and this release is known to stimulate the formation of new mineral tissue. The release of silicon, in the form of orthosilicic acid is known to play a crucial role as a stimulus to induce osteogenesis. The composition of bioactive glasses can be tuned to incorporate LiO₂, which yields typically up to around 5% wt of Li ions in SiO₂ bioactive glasses. In this way bioactive glasses can incorporate the release of lithium alongside their other constituents. The problems with this approach are the poor loading degree of lithium and poor and determined biodegradability of the bioactive glass, which limits the possibility to freely tune the lithium amount and release rate, which determines the amount of stimulus given. In addition, the inevitable release of phosphorous has been connected to cytotoxicity of bioactive glasses.

Alternative methods for controlled co-delivery delivery of lithium and orthosilicic acid for regenerative purposes are therefore desirable.

### SUMMARY OF THE INVENTION

The present invention provides lithiated porous silicon particles comprising lithium, silicon and oxygen.

The present invention also provides a composition comprising the lithiated porous particles herein described.

The present invention also provides lithiated porous silicon particles for use in therapy, wherein the particles comprise lithium and silicon.

The present invention also provides lithiated porous silicon particles for use in the repair or regeneration of tissue, such as mineralised tissue or soft tissue, wherein the particles comprise lithium and silicon.

The present invention also provides lithiated porous silicon particles for use in the repair or regeneration of mineralised tissue, preferably mineralised bone or dental tissue, wherein the particles comprise lithium and silicon.

The present invention also provides lithiated porous silicon particles for use in the prevention or treatment of dental cavities, bone fractures, caries, periodontitis, alveolar bone loss, craniofacial defects (e.g. cleft palate/alveolar ridge), bone loss, dentine loss, and surgical bone removal, wherein the particles comprise lithium and silicon.

The present invention also provides a method of preparing lithiated porous silicon particles, the method comprising contacting porous silicon particles with a source of lithium in the presence of oxygen.

Throughout the description and claims of this specification, the words "comprise" and "contain" and variations of the words, for example "comprising" and "comprises", mean "including but not limited to", and do not exclude other components, integers or steps. Moreover the singular encompasses the plural unless the context otherwise requires: in particular, where the indefinite article is used, the specification is to be understood as contemplating plurality as well as singularity, unless the context requires otherwise.

Preferred features of each aspect of the invention may be as described in connection with any of the other aspects. Within the scope of this application it is expressly intended that the various aspects, embodiments, examples and alternatives set out in the preceding paragraphs, in the claims and/or in the following description and drawings, and in particular the individual features thereof, may be taken independently or in any combination. That is, all embodiments and/or features of any embodiment can be combined in any way and/or combination, unless such features are incompatible.

### BRIEF DESCRIPTION OF THE DRAWINGS

One or more embodiments of the invention will now be described, by way of example only, with reference to the accompanying drawings, in which:
Figure 1 is a series of graphs showing the release profile of lithium and silicon from lithiated porous silicon (LipSi) particles, and from porous silicon particles, over time;
Figure 2A depicts the X-Ray Diffraction (XRD) diffractograms of the LipSi particles and porous silicon particles;
Figure 2B shows the XRD peak assignment for one embodiment of the invention and reference materials comprising lithium and silicon;
Figure 3 shows magic angle spinning nuclear magnetic resonance (MAS-NMR) spectra of isotopes ⁷Li and ²⁹Si measured from sample SS21.
Figure 4 is a series of high-resolution transmission electron microscopy (HRTEM) images of LipSi particles and porous silicon particles;
Figure 5 is a graph showing the position of the silicon Raman spectroscopic peak for various LipSi, pSi, and silicon samples;
Figure 6 shows hydrodynamic diameter of LipSi and pSi nanoparticles measured with dynamic light scatterning (DLS) (a) and the elemental composition of the particles as detected by TOF-SIMS (b);
Figure 7 is a series of graphs showing the in vitro biological activity of lithium released from LipSi particles. Figures 6a and 6b show the change in Axin2 expression from cells exposed to LipSi particles (Li/Si ratio mentioned in brackets) compared to 25mM LiCl and medium only; Figure 6c shows the normalised viability of cells treated with LipSi particles compared to porous silicon particles only and medium only. Statistical test done with one-way Anova. Notation: non significant (ns); p<0.05 (*); p<0.01 (**); p<0.001 (***); p<0.0001;
Figure 8 is two micro computed tomography (micro-CT) images showing the formation of mineralised tissue at 4 weeks following LipSi particle treatment on an *in vivo* exposed pulp cavity dental model (a). Control image for animal treated with injury but no particles (b);
Figure 9 shows results of a transmission electron microscopy-electron energy loss spectroscopy (TEM-EELS) study of pSi and LipSi particles. Figure 9A shows the map of relative concentration of silicon and lithium within a porous silicon and a lithiated porous silicon particle. Figure 9B is a series of electron energy loss spectra at the lithium and silicon absorption edges. Figure 9C is to graphs showing the relative silicon and lithium concentration as well as the particle thickness along a line through the porous silicon and lithiated porous silicon particles.
Figure 10 is a series of graphs showing nitrogen sorption curves for pSi and LipSi particles;
Figure 11 is a series of graphs showing qPCR analysis results for gene activation (RUNX2 - Figure 11A; CAP - Figure 11B; BMP2 - Figure 11C; OCN - Figure 11D) following exposure of primary human periodontal stem cells to different media;
Figure 12 is a series of micro computed tomography (micro-CT) images showing murine mandible at 2 weeks following treatment on an *in vivo* mouse model of periodontal disease;
Figure 13 is a series of graphs showing the bone mineral density (BMD) and bone volume over total volume (BV/TV) in an *in vivo* mouse model of periodontal disease. Figure 13A shows BMD 2 weeks after treatment; Figure 13B shows BV/TV 2 weeks after treatment; Figure 13C shows BMD 6 weeks after treatment; Figure 13D shows BV/TV 6 weeks after treatment;
Figure 14 is a series of histological images showing the effect of control (1st row), LipSi (2^{nd} row), LiCl (3^{rd} row), F127 carrier (4^{th} row) and pSi (5^{th} row) treatment on murine mandibular samples (overview of sample - 1^{st} column; bone - 2^{nd} column; cementum - 3^{rd} column; ligament - 4^{th} column) at two weeks of treatment.

### DETAILED DESCRIPTION

The present invention provides lithiated porous silicon (LipSi) particles comprising lithium, silicon and oxygen. The porous particles comprising lithium and silicon advantageously allow for the sustained release of lithium in a biological environment. Advantageously, the particles may release a therapeutically effective amount of lithium under physiological conditions.

Orthosilicic acid (Si(OH)₄) is also released from the particles in a sustained manner in a biological environment. Lithium is a dual-inhibitor of GSK-3 activity and thus the present particles have potential applications in the treatment of conditions which can be prevented or improved by GSK-3 inhibition. Furthermore, both lithium and orthosilicic acid have mineral tissue regeneration/repair properties. Thus the particles of the present invention conveniently deliver two mineralised tissue regeneration agents in a single composition. The particulate form enables ready incorporation of the product into many useful compositions and formulations, e.g. into bone and dental cements, whilst still maintaining the desired controlled release of lithium (and orthosilicic acid). The porous (e.g. mesoporous) silicon structure is beneficial as it is necessary to allow the degradation/dissolution of the material on biologically relevant timescales and to tune the kinetics of degradation/dissolution. It may also be desirable to load another type of drug into the pores and thus use the particles to deliver a further drug along with the lithium and silicic acid.

Advantageously, the particles may release a therapeutically effective amount of lithium under physiological conditions. Advantageously, the particles may release a therapeutically effective amount of silicon (e.g. in the form of silicic acid) under physiological conditions. The particles preferably provide sustained release of lithium and/or silicon under physiological conditions. The particles may preferably release lithium (e.g. a therapeutically effective amount of lithium) under physiological conditions over at least 6 hours, more preferably over at least 12 hours and still more preferably over at least 24 hours. The particles may preferably release lithium (e.g. a therapeutically effective amount of lithium) under physiological conditions over at least 1 day, over at least 2 days, over at least 3 days, over at least 4 days, over at least 5 days, over at least 6 days, over at least 7 days, over at least 10 days or over at least 14 days. The particles may preferably release lithium (e.g. a therapeutically effective amount of lithium) under physiological conditions over from 6 hours to 21 days, from 12 hours to 14 days or from 24 hours to 7 days. The particles may preferably release silicon (e.g. a therapeutically effective amount of silicon) under physiological conditions over at least 6 hours, more preferably over at least 12 hours and still more preferably over at least 24 hours. The particles may preferably release silicon (e.g. a therapeutically effective amount of silicon) under physiological conditions over at least 1 day, over at least 2 days, over at least 3 days, over at least 4 days, over at least 5 days, over at least 6 days, over at least 7 days, over at least 10 days, over at least 14 days or over at least 21 days. The particles may preferably release silicon (e.g. a therapeutically effective amount of silicon) under physiological conditions over from 6 hours to 21 days, from 12 hours to 14 days or from 24 hours to 7 days. Preferably silicon is released in the form of silicic acid.

"Porous silicon" is a term well accepted in the field as referring to a material comprising a continuous silicon skeleton with interconnected pores, generally obtained from a top-down subtractive process of etching silicon. Thus, in the lithiated porous silicon particles of the present invention, each particle preferably comprises a single continuous silicon skeleton, with lithium and oxygen incorporated therein. The lithiated porous silicon particles of the present invention preferably each comprise (more preferably consist of) a continuous skeleton of silicon (e.g. crystalline silicon) permeated by lithium and oxygen to generate amorphous and crystalline domains of lithium silicates and lithium silicides. Thus whilst the particles of the present invention preferably comprise different domains (e.g. crystalline silicon, lithium silicates, lithium silicides etc), the particles are not composite particles. Each particle is thus preferably monolithic. Each particle is thus preferably not comprised of multiple smaller particles. This is a function of the process used to synthesise the particles, as described in further detail below. The lithiated porous silicon particles of the present invention are thus fundamentally different in structure, properties and composition compared to particles produced by sintering or otherwise joining smaller silicon (or a silicon-containing material e.g. silicon monoxide) particles together. The lithiated porous silicon particles of the present invention comprise lithium, silicon and oxygen. The particles may preferably comprise at least 0.001 wt%, at least 0.01 wt%, at least 0.1 wt%, at least 0.5 wt%, at least 1 wt%, at least 5 wt% or at least 10 wt% lithium. The particles may preferably comprise up to 65 wt%, up to 60 wt%, up to 55 wt%, up to 50 wt%, up to 40 wt %, up to 30 wt%, up to 20 wt%, up to 10 wt% lithium or up to 5 wt% lithium. The particles may preferably have a lithium content of from about 0.001 wt% to about 65 wt%, about 0.001 wt% to about 55 wt%, about 0.1 wt% to about 50 wt%, about 1 wt% to about 40 wt%, or about 2 wt% to about 25 wt%, e.g. from about 5 wt% to about 10 wt%.

The particles may preferably comprise at least 10 wt%, at least 20 wt%, at least 30 wt%, at least 35 wt%, at least 40 wt%, at least 45 wt%, at least 50 wt%, at least 60 wt%, at least 70 wt% or at least 80 wt% silicon. The particles may preferably comprise up to 95 wt%, up to 90 wt%, up to 80 wt%, up to 70 wt%, up to 60wt%, up to 50 wt% or up to 40 wt% silicon. The particles may preferably have a silicon content of from about 10 wt% to about 95 wt%, about 20 wt% to about 90 wt%, about 25 wt% to about 80 wt%, or about 30 wt% to about 75 wt%, e.g. from about 40 wt% to about 60 wt%.

The presence of oxygen in the lithiated porous silicon particles of the present invention may advantageously passivate the material, e.g. by forming oxides such as a protective oxide coating, thereby giving the particles relative stability to air and moisture (e.g. rendering the material non-pyrophoric) and making it suitable for use in biological environments. The particles may preferably comprise at least 10 wt%, at least 20 wt%, at least 30 wt%, at least 35 wt%, at least 40 wt%, at least 45 wt% or at least 50 wt% oxygen. The particles may preferably comprise up to 85 wt%, up to 80 wt%, up to 70 wt%, up to 60 wt% or up to 50 wt% oxygen. The particles may preferably have an oxygen content of from about 5 wt% to about 85 wt%, about 5 wt% to about 75 wt%, about 10 wt% to about 70 wt%, or about 20 wt% to about 65 wt%, e.g. from about 30 wt% to about 60 wt%.

The lithiated porous silicon particles of the present invention may preferably have a greater proportion of oxygen at or close to the surface of the particles (as compared to the bulk). The lithiated porous silicon particles of the present invention may preferably have a surface oxygen content of at least 20 atm%, at least 30 atm%, at least 40 atm%, at least 50 atm%, at least 60 atm%, at least 70 atm%, or at least 80 atm% oxygen. The surface of the particles may preferably have a surface oxygen content of up to 98 atm%, up to 95 atm% or up to 90 atm% oxygen. The particles may preferably have a surface oxygen content of from about 20 atm% to about 95 atm%, about 30 atm% to about 94 atm%, about 40 atm% to about 93 atm% or about 50 atm% to about 90 atm%.

The lithiated porous silicon particles of the present invention may advantageously contain a relatively high proportion of lithium relative to silicon, for example as measured by the %weight ratio of lithium to silicon (Li/Si) in the particles, e.g. as measured by ICP-MS as set out in the examples below. A Li/Si ratio of 100 wt% would mean that the particles contain an equal weight of Li and Si. A Li/Si ratio of 50 wt% would mean that the particles contain half as much lithium by weight as silicon. The lithiated porous silicon particles of the present invention may preferably have a Li/Si ratio of at least 0.1 wt%, at least 0.2 wt%, at least 0.5 wt%, at least 1 wt%, at least 5 wt%, at least 10 wt%, at least 20 wt%, at least 30 wt%, at least 40 wt% or at least 50 wt%. The lithiated porous silicon particles of the present invention may preferably have a Li/Si ratio of up to 100 wt%, up to 90 wt%, up to 80 wt% or up to 75 wt%. The lithiated porous silicon particles of the present invention may preferably have a Li/Si ratio of from about 0.1 wt% to about 100 wt%, from about 0.2 wt% to about 95 wt%, from about 0.5 wt% to about 90 wt%, from about 1 wt% to about 85 wt%, from about 5 wt% to about 80 wt% or from about 10 wt% to about 75 wt%.

The lithium, silicon and oxygen content of the particles can be controlled by varying the reaction conditions/parameters of their production, for example the Li:Si ratio, oxygen concentration, temperature, reaction time, ambient gas, porosity, pore size, etc. The overall lithium, silicon and oxygen content of the particles can be measured by XPS following etching, for example following argon etching as described in the examples below. The surface composition of the particles can be measured by XPS (without etching) e.g. as described in the examples below.

Preferably, the particles comprise crystalline silicon. The crystalline nature of silicon within the particles may be verified by X-ray diffraction (XRD), e.g. as described in the examples below. The presence of a crystalline silicon phase or phases is beneficial as it allows for the incorporation of lithium within the silicon crystal structure (e.g. as interstitial lithium). Thus, the particles of the present invention preferably comprise lithium embedded within a silicon matrix/crystal structure. It is believed that at least some of the lithium is present as Li⁺ cations. Advantageously, the incorporation of lithium within the crystal structure of the silicon, rather than, for example, lithium simply residing in the pores of porous silicon, allows for greater control over its release, providing sustained release formulations. Furthermore, it is known that the silicon matrix can contain lithium to over 50 wt% (the known alloy Li₂₂Si₅ has a lithium content of 52 wt%). Thus, by incorporating lithium within the silicon matrix high loading of lithium can be obtained, for example as compared to lithium-doped bioactive glasses which have a much lower maximum lithium content. High levels of lithium incorporation mean that higher quantities of therapeutic lithium can be delivered using the present particles.

The incorporation of lithium inside the crystal matrix of silicon enables slow and tightly controlled release of the lithium via dissolution of the material or slow diffusion from the material.

The lithiated porous silicon particles of the present invention may preferably comprise an amorphous phase. The amorphous phase may comprise one or more amorphous silicon oxides and optionally lithium. The particles may comprise an amorphous phase in addition to a crystalline silicon phase as well as optionally in addition to other phases. The particles may alternatively be entirely amorphous. The presence of an amorphous phase may be identified by XRD, e.g. as described in the examples below. The composition of the amorphous phase may be verified by solid state NMR of Li and Si as known in the art. The presence of one or more amorphous phases is advantageous because it confers greater solubility (in aqueous environments) to the lithium components in the particles. Increased solubility leads to greater bioavailability of the lithium (and orthosilicic acid) when used in vivo.

The lithiated porous silicon particles of the present invention may preferably comprise one or more lithium silicates, for example having the formula Li_{w}SiₓO_{y}. Lithium silicates are formed by the oxidation of the lithium/silicon material during or after the formation of the lithiated porous silicon particles. The porous particles of the present invention may comprise Li₂SiO₃. The porous particles of the present invention may comprise Li₂Si₂O₅. The porous particles of the present invention may comprise Li₄SiO₄. The presence of these, and other, lithium silicates, may be identified using XRD and solid state NMR, e.g. as described in the examples below.

The lithiated porous silicon particles of the present invention may preferably comprise one or more lithium silicides, for example having the formula Li_{w}Siₓ. Examples of lithium silicides include Li₁₃Si₄, Li₂₂Si₅, Li₇Si₃ and Li₁₂Si₇, Li_{4.4}Si and Li₁₂Si₇. The presence of these, and other, lithium silicides, may be identified using XRD and solid state NMR, e.g. as described in the examples below.

The lithiated porous silicon particles of the present invention may preferably comprise one or more lithium oxides. These may be in the form of lithium silicates, as described above, or may be of the formula LiₓO_{y}, for example Li₂O. The presence of lithium oxides may be identified using XRD and solid state NMR, e.g. as described in the examples below.

The lithiated porous silicon particles of the invention may preferably comprise one or more silicon oxides, for example silicon dioxide (SiO₂) and sub-oxides (SiOₓ). The presence of oxidised species, particularly at the surface of the particles, provides a protective layer, increasing the stability of the particles. The presence of silicon oxides may be identified using XRD and solid state NMR, e.g. as described in the examples below.

The lithiated porous silicon particles of the present invention may be of any size but are preferably microparticles, nanoparticles or a mixture thereof. The particles of the invention may preferably have an average particle diameter of from about 1 nm to about 5 mm, more preferably from about 10 nm to about 1 mm, and still more preferably from about 100 nm to about 500 µm. The particles may preferably be microparticles with an average diameter of from about 1 µm to about 1000 µm, from about 5 µm to about 500 µm or from about 100 µm to about 200 µm. Alternatively, the particles may preferably be nanoparticles with an average diameter of from about 50 nm to about 900 nm, from about 100 nm to about 700 nm or from about 150 nm to about 600 nm.

The lithiated porous silicon particles of the present invention are porous, and may have an average porosity of from about 5% to about 90%, from about 10% to about 90%, from about 15% to about 90%, or from about 20% to about 90%, e.g. from about 25% to about 75% or from about 30% to about 65%. The lithiated porous silicon particles of the present invention may have an average porosity of greater than 5%, greater than 10%, greater than 25%, or greater than 50%. Porosity may be measured with nitrogen sorption analysis, as is known in the art. The porosity and pore size of the LipSi particles can be tuned during the production of the porous silicon starting material, which can be made at up to 90% porosity (and higher). Without wishing to be bound by theory, it is believed that insertion of lithium into the porous silicon during the lithiation process leads to some reduction in pore size and porosity, with incorporation of greater amounts of lithium leading to a greater reduction in pore size and porosity. It is expected that particles containing relatively low amounts of lithium (e.g. up to about 0.1 atm% lithium) would have roughly the same pore size and porosity as the porous silicon starting material. Advantageously, controlling the porosity of the porous silicon (and thus the product) controls the dissolution profile of the product. Higher porosity leads to faster dissolution of the product. Higher porosity silicon may also allow incorporation of a greater proportion of lithium into the product.

The lithiated porous silicon particles may preferably be mesoporous (e.g. may have an average pore size of from about 2 nm to about 50 nm). The particles may preferably have an average pore size (average pore diameter) of no more than about 200 nm, no more than about 100 nm, no more than about 50 nm, no more than about 25 nm, no more than about 20 nm, no more than about 15 nm, no more than about 10 nm, from about 1 nm to about 200 nm, from about 1 nm to about 100 nm, from about 1 nm to about 50 nm, from about 1 nm to about 20 nm, or from about 1 nm to about 10 nm, e.g. from about 5 nm to about 25 nm. The lithiated porous silicon particles may preferably have an average pore size of greater than about 1 nm, greater than about 2 nm, greater than about 3 nm, greater than about 4 nm or greater than about 5 nm. Pore size may be measured using Barrett-Joyner-Halenda (BJH) analysis, as is known in the art.

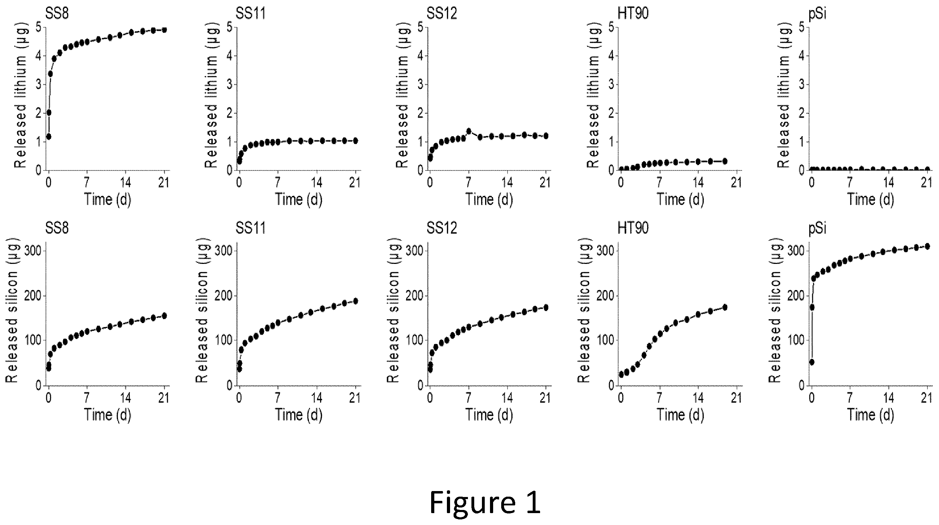

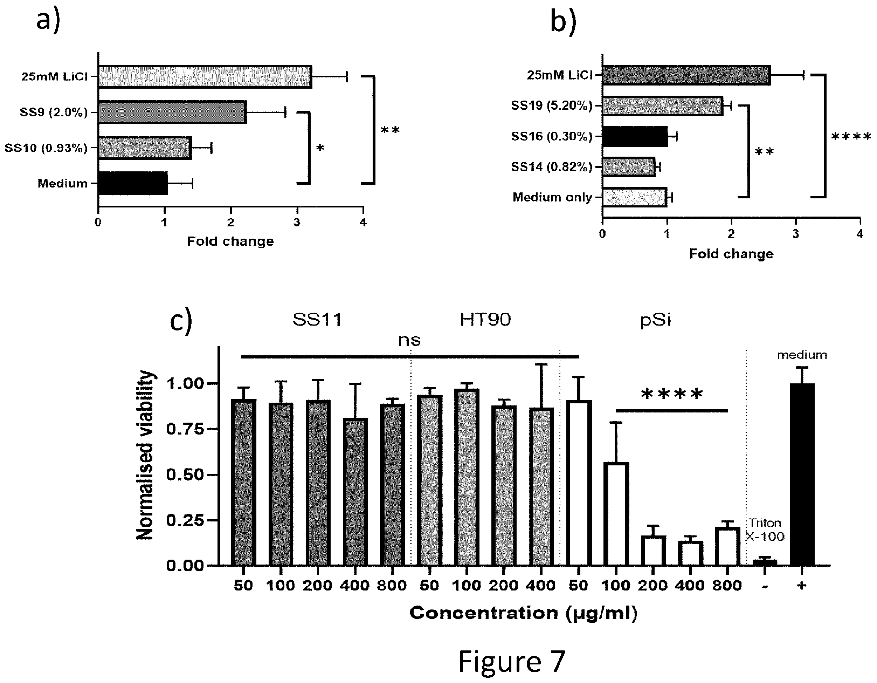

The lithiated porous silicon particles of the present invention may advantageously possess one or more of the following properties:
- biocompatible (e.g. as measured using MTT assay, or ISO tests for biocompatibility e.g. ISO 10993-5:2009). The biocompatiblity of the LipSi particles of the present invention is demonstrated in the examples below, e.g. Example 11 and Figure 7;
- bioresorbable (e.g. as measured using USP dissolution testing). Bioresorbable refers to materials that are broken down and absorbed by the body. The bioresorbability of a material may be tested by its dissolution rate in a simulated physiological environment. In particular, the lithiated porous silicon particles of the present invention have a controlled dissolution kinetic in 50 mM phosphate buffer at pH 7.4 in sink condition. Figure 1 and shows that the dissolution rate/kinetic can be controlled between 14-77% at 1 day, 16-81% at 3 days, 23-88% at 7 days and 28-94% at 14 days;
- air/oxygen/water stable (e.g. as measured using XPS + XRD following exposure to the desired condition). Stability may be defined as lack of exchange of energy with the environment over time and thus may be measured using differential thermal analysis (DTA), differential scanning calorimetry (DSC) or isothermal microcalorimetry (IMC);
- non-pyrogenic and/or non-pyrophoric
- provide sustained release of Li/Si (e.g. in the form of Li⁺ ions/orthosilicic acid)
- promote an increase in Axin2 expression (e.g. as measured by incubating cells with LipSi particles or their degradation product, extracting RNA, doing reverse transcription of RNA to cDNA and measuring the relative concentration of Axin2 cDNA sequences with RT-qPCR);
- induce osteogenesis (e.g. as measured by testing osteogenic markers (e.g. ALP, OSX, OCN, OPN, RUNX2, BMP2) with the same qPCR method as described for Axin2 above);
- regenerate/repair mineralised tissue (e.g. as measured in vitro with Alizarin Red staining or in vivo with µCT)
- regenerate/repair connective tissue (e.g. as measured in vivo by histological analysis)

Particularly, the lithiated porous silicon particles of the present invention are suitable for use in aqueous environments (e.g. in biological environments). This is in contrast to many materials developed for use in battery technology which are generally suitable for use only in non-aqueous (and usually inert) environments. Exposure of these battery materials to aqueous environments or even to air/oxygen is likely to result in combustion of the material. Thus, the lithiated silicon particles of the present invention advantageously do not combust (i.e. do not catch fire) upon contact with water, air or oxygen.

The present invention also provides a composition (e.g. a pharmaceutically acceptable composition) comprising the porous particles hereinbefore described. The composition may be in the form of a solid (e.g. a powder), a semi-solid, a suspension, a solution, a putty, a clay or a gel. The composition may comprise the porous particles hereinbefore described and a carrier. The porous particles may be dispersed in a carrier.

The particles and compositions of the present invention are particularly suitable for use in dental and bone cements, due to their mineralised tissue repair/regeneration properties. Thus, the present invention also provides a dental cement comprising the particles or compositions hereinbefore described. The present invention also provides a bone cement comprising the particles or compositions hereinbefore described.

The porous particles and compositions of the present invention may also be used as a preventative measure, for example against bone/dentine loss. Particularly suitable formulations for preventative use include mouthwash, toothpaste and dental floss. Thus, the present invention also provides a mouthwash, toothpaste or dental floss comprising the particles or compositions hereinbefore described.

The particles and compositions of the present invention may be formulated for delivery in any suitable form, including:
- dispersed nano/microparticles for local injection (e.g. periodontal ligament)
- microparticles for incorporation within dental and bone cements
- concentrated particle putty/clay for moulding within defects
- films for patches, implantables and tablets
- particle-polymer composites to use either as they are or as a feedstock material for device manufacturing (e.g. 3D printing or dental floss)
- dispersed nano/microparticles particles for mouthwash or toothpaste
- blending with polymers and making feedstock/filaments for additive manufacturing
- natural and synthetic, degradable and non-degradable polymeric membranes containing the particles

As described above, and demonstrated in the examples below, the lithiated porous silicon particles of the present invention provide the sustained release of lithium and orthosilicic acid, both of which have therapeutic properties.

The present invention also provides lithiated porous silicon particles for use in therapy, wherein the particles comprise lithium and silicon. The lithiated porous silicon particles for use in therapy may preferably be as hereinbefore defined with respect to the lithiated porous silicon particles of the invention.

The lithiated porous silicon particles for use according to the present invention may preferably be used in the prevention or treatment of any condition susceptible of being improved or prevented by GSK-3 inhibition. The particles provide the sustained release of lithium, which is a known GSK-3 inhibitor. It is therefore to be expected that the particles will be therapeutically effective in preventing or treating conditions susceptible of being improved or prevented by GSK-3 inhibition.

The lithiated porous silicon particles for use according to the present invention may be used in the repair or regeneration of tissue, such as mineralised tissue or soft tissue. The tissue may preferably be bone or dental tissue, such as dentine, alveolar bone, periodontal ligament, cementum, temporal bone or skeletal bone tissue.

The lithiated porous silicon particles for use according to the present invention may preferably be used in the repair or regeneration of mineralised tissue, preferably mineralised bone or dental tissue. Advantageously, the particles provide sustained release of lithium and orthosilicic acid, both of which are known to stimulate the regeneration and repair of mineralised tissue.

The mineralised tissue may preferably be mineralised bone tissue. Alternatively the mineralised tissue may preferably be mineralised dental tissue. Specific types of mineralised bone/dental tissue include, but are not limited to, dentine, alveolar bone, periodontal ligament, cementum, temporal bone, skeletal bone tissue. Preferred types of mineralised bone/dental tissue include, but are not limited to, dentine, alveolar bone, cementum, temporal bone, skeletal bone tissue.

The lithiated porous silicon particles for use according to the present invention may preferably be used in the repair or regeneration of soft tissue, preferably dental soft tissue, e.g. periodontal ligament.

The lithiated porous silicon particles for use according to the present invention may preferably be used in the prevention or treatment of dental cavities, dental fissure, bone fractures, caries, periodontitis, periodontal ligament disorders, alveolar bone loss, craniofacial defects (e.g. cleft palate/alveolar ridge), bone loss, dentine loss, spinal cord injury and surgical bone removal (e.g. due to cancer or other pathologies, fractures or congenital defects).

The lithiated porous silicon particles for use according to the present invention may also be used in the prevention or treatment of other conditions susceptible of being improved or prevented by GSK-3 inhibition. Such conditions include, but are not limited to bipolar disorder, Alzheimer's disease, type 2 diabetes mellitus, apoptosis in glioma and pancreatic cancer. The lithiated porous silicon particles for use according to the present invention may also be used in some immunotherapies.

The lithiated porous silicon particles of the present invention may also be used in in vitro applications. For example, they may be used to stimulate cells in culture. The stimulated cells may then be used in vivo, or in culture for assays or other molecular biology work.

The present invention also provides a kit comprising lithiated porous silicon particles comprising lithium and silicon, together with instructions for the use thereof in the prevention or treatment of any condition susceptible of being improved or prevented by GSK-3 inhibition (e.g. the regeneration or repair of mineralised tissue). The lithiated porous silicon particles may preferably be as hereinbefore defined. The condition may be any condition hereinbefore defined.

The present invention also provides a method of preparing lithiated porous silicon particles. The method comprises contacting porous silicon particles with a source of lithium in the presence of oxygen.

The method provides lithiated porous silicon particles which dissolve in a controlled fashion in biological environments. This provides sustained release of lithium (and orthosilicic acid) in biological environments, e.g. for regeneration of mineralised tissue. The particulate form enables incorporation of the product into many useful formulations, e.g. into bone and dental cements, whilst still maintaining the desired controlled release of lithium (and orthosilicic acid). Despite the high reactivity of lithium sources and many lithiated products (e.g. lithiated battery materials) the method usefully provides relatively stable particles that can be exposed to air and moist environments without pyrophoric reactions. Furthermore, the method does not require the use of toxic additives, and it yields a biocompatible product.

The method of the present invention is carried out in the presence of oxygen, i.e. O₂. The oxygen may be in the form of oxygen gas (e.g. when the method is carried out in a gaseous environment, such as in the solid state method described below) or may be dissolved oxygen (e.g. when the method is carried out in an aqueous environment, such as in the hydrothermal method described below). Preferably, oxygen is present in at least 5, at least 10, at least 25, at least 50 or at least 100 parts per million (ppm). Oxygen may be present in at least 200, at least 500, or at least 1000 ppm. Oxygen may be present in up to 250,000 ppm, up to 100,000 ppm, up to 10,000 ppm, up to 5000 ppm, up to 1000 ppm or up to 500 ppm. Oxygen may be present in from 5 ppm to 250,000 ppm, from 10 ppm to 100,000 ppm, from 20 ppm to 50,000 ppm or from 50 ppm to 1000 ppm. In some cases it may be preferable to carry out the method of the invention in an oxygen enriched atmosphere, in which case higher levels of oxygen may be present. Thus, oxygen may alternatively be present in up to 1,000,000 ppm, up to 750,000 ppm or up to 500,000 ppm.

The method may be carried out in the presence of water. Water may preferably be present in at least 1, at least 2, at least 3, at least 4 or at least 5 ppm. Water may be present in at least 10, at least 25, at least 50 or at least 100 ppm.

It is believed that the presence of oxygen and/or water leads to the formation of oxide species in the product (e.g. lithium oxides, silicon oxides, lithium silicates), particularly at or near the surface of the particles, which decreases the reactivity of the particles relative to particles formed in strictly oxygen and water-free conditions (such as materials developed for battery applications). This helps produce a relatively stable product that is non-pyrophoric, and suitable for use in biological applications. As a comparison the oxygen concentration in state-of-the-art gloveboxes for lithium batteries is typically under 1 ppm and the H₂O concentration is as close to 0 ppm as possible.

Thus preferably at least part of the oxygen in the lithiated porous silicon particles of the invention originates from oxygen or water, preferably oxygen, in the atmosphere in which the method of the present invention is carried out.

The method of the invention may be carried out in a substantially non-oxidising/inert atmosphere (e.g. under nitrogen or argon), provided that at least some oxygen is present. Controlling or limiting the amount of oxygen present may control the degree of oxidation in the product, thereby helping to control its dissolution characteristics and thus the availability and release rate of lithium and silicic acid from the particles in a biological environment. Alternatively the method may be carried out in air. Surprisingly the inventors have found that it is possible to form a useful product in this way, whereas in general lithiation processes are carried out in a strictly oxygen and air-free atmosphere due to the high reactivity of lithium species, which may be expected to render such processes and their products pyrophoric. The exposure to oxygen during the lithiation advantageously leads to a degree of oxidation in the product which, without wishing to be bound by theory, it is believed forms a protective layer, providing a relatively air stable product.

The present invention utilises porous silicon in particulate form. This provides a particulate product, i.e. lithiated porous silicon particles are generated. The particles may be of any desired size but preferably the porous silicon particles are microparticles, nanoparticles or a mixture thereof.

Nanoparticles may be defined as particles ranging in size (e.g. having an average dimeter of) from about 10 nm to about 1000 nm. Microparticles may be defined as particles ranging in size (e.g. having an average dimeter of) from about 1 µm to about 1000 µm. The size of the nanoparticles may, for example, be measured using dynamic light scattering methods and the size of microparticles or mixture of sizes may, for example, be measured using laser diffraction.

The suitable particle size strongly depends on the end application for the particles. If the delivery site is small such as a periodontal ligament and/or the particles need to be injectable, then nanoparticles or small microparticles (e.g. an average diameter of less than 10 micron) are preferred. In major bone regeneration, the size can be bigger. For major bone regeneration size can be highly polydisperse including particles up to hundreds of microns.

In the method of the present invention the porous silicon preferably has an average particle diameter of from about 1 nm to about 5 mm, more preferably from about 10 nm to about 1 mm, and still more preferably from about 100 nm to about 500 µm. The porous silicon particles may preferably be microparticles with an average diameter of from about 1 µm to about 1000 µm, from about 5 µm to about 500 µm or from about 100 µm to about 200 µm. Alternatively, the porous silicon particles may preferably be nanoparticles with an average diameter of from about 50 nm to about 900 nm, from about 200 nm to about 800 nm or from about 250 nm to about 750 nm.

Preferably, the method of the present invention provides lithiated porous silicon particles that are in the form of microparticles, nanoparticles or a mixture thereof. The size of lithiated porous silicon particles can be controlled in various ways. The primary factor in determining the particle size of the lithiated porous silicon product is the particle size of the porous silicon starting material. The particle size of the porous silicon starting material can be controlled by the conditions of silicon etching used to produce the particles. The particle size can be further adjusted using particle size reduction methods (e.g. ball-milling, grinding, sonicating) before or after lithiation. Size separation methods (e.g. through centrifuging or filtering) may be utilized to select the desired final size fraction, before or after lithiation. Using a porous silicon starting material with nano dimensions in the method can be expected to result in lithiated porous silicon nanoparticles. Using a porous silicon starting material with micron scale dimensions in the method would be expected to result in lithiated porous silicon microparticles, or nanoparticles where further size reduction steps are utilised. The size of the lithiated porous silicon particles formed by the method can, thus, be readily controlled by any combination of controlling the dimensions of the porous silicon particle starting material, the size reduction parameters, and by size selection.

The method of the present invention may be carried out (e.g. the porous silicon and the source of lithium may be contacted together) at any appropriate temperature to form the desired lithiated porous silicon product. However, the porous silicon and the source of lithium are preferably heated above room temperature (e.g. to at least 50 °C, at least 100 °C or at least 200 °C), preferably to a temperature of from about 50 °C to about 1000 °C.

In the method of the invention, porous silicon particles are contacted with a source of lithium. The silicon to lithium molar ratio in the starting materials may be varied, for example to produce varying levels of lithiation in the LipSi particle product. Preferably, the molar ratio of silicon to lithium is from about 10:1 to about 1:10, or from about 5:1 to about 1:5. More preferably, the molar ratio of silicon to lithium is from about 1:1 to about 1:10, or from about 1:2 to about 1:5, for example about 2:3, about 1:2, about 3:7, about 1:3, about 1:4, or about 1:5. Advantageously, the use of a higher proportion of lithium enables the incorporation of greater amounts of lithium within the porous silicon, leading to higher loading of lithium and thus higher release and efficacy of the LipSi particles so produced. The desired loading of lithium in the product depends on the end application and tuning the molar ratio of silicon to lithium in the method allows different lithium loadings to be achieved. The method thus allows the tuning of the ratio of released ions, silicon and lithium, which is one of the benefits of LipSi particles compared to bioactive glass.

The method of the invention utilises a source of lithium (a lithium source). Any suitable lithium source may be used, for example in elemental (metallic) form or a lithium salt. Preferably a lithium salt is used. Lithium salts may be organic or inorganic. Examples of suitable inorganic lithium salts include halides (e.g. lithium bromide, lithium chloride, lithium iodide, lithium fluoride), hydroxide (LiOH), carbonate (Li₂CO₃), oxide (LiO₂), peroxide (Li₂O₂), nitrate, sulphate, phosphate, and mixtures thereof, preferably lithium chloride (LiCl), lithium hydroxide (LiOH) or lithium carbonate (Li₂CO₃). Salts may be anhydrous or in the form of a hydrate, e.g. LiOH.H₂O. Examples of suitable organic lithium salts include citrate (Li₃C₆H₅O₇). Preferably, the source of lithium is selected from metallic lithium, LiCl, LiOH, LiOH.H₂O, Li₂CO₃, lithium citrate (Li₃C₆H₅O₇), and mixtures thereof, more preferably LiCl, LiOH, LiOH.H₂O, Li₂CO₃, lithium citrate (Li₃C₆H₅O₇), and mixtures thereof, e.g. LiCl, LiOH or Li₂CO₃. Preferably, the lithium source does not contain silicon. Preferably, the lithium source is not a lithium silicate. Advantageously the method of the present invention can be carried out using non-toxic lithium salts and other starting materials. For example, highly toxic lithium salts, such as those used in battery technology (e.g. LiPF₆, LiBF₄,) can be avoided. The inventors have surprisingly found that lithiation of porous silicon can be achieved without the use of harmful, cytotoxic additives (e.g. ethylene carbonate, dimethyl carbonate and diethyl carbonate) and thus a pharmaceutically acceptable, biocompatible product can be made. Despite the high reactivity of lithium species, the method of the invention surprisingly provides a stable (e.g. air stable) product.

The method of the present invention uses porous silicon as a starting material. Preferably, the porous silicon has an average porosity of from about 5% to about 90%, from about 10% to about 90%, from about 15% to about 90%, or from about 20% to about 90%, e.g. from about 25% to about 75% or from about 30% to about 65%. The porous silicon starting material may have an average porosity of greater than 5%, greater than 10%, greater than 25%, or greater than 50%. Porosity of the porous silicon starting material may be controlled by varying certain parameters in its synthesis, for example by controlling the resistivity of the silicon, the concentration of hydrofluoric acid and oxidant in the etching solution and metal precursor concentration in metal assisted chemical etching and the voltage or current during anodic etching, as described in US 8,568,877 and US 5,358,600.

Porosity may be measured using Brunauer-Emmett-Teller (BET) analysis, as is known in the art. Advantageously, controlling the porosity of the porous silicon (and thus the product) controls the dissolution profile of the product. Higher porosity leads to faster dissolution of the product. Higher porosity silicon also allows incorporation of a greater proportion of lithium into the product. Without wishing to be bound by theory, it is believed that this is because the Li only penetrates to a shallow depth into the crystal structure, so a shallow porous silicon skeleton, for example, allows for greater lithium incorporation. The nature of the typically long interconnected pores of porous silicon means there is a large exposed surface area which is enabling for tailoring the dissolution kinetics, which is advantageous for controlling the release of lithium from the product.

The porous silicon may preferably be mesoporous (e.g. may have an average pore size of from about 2 nm to about 50 nm). The porous silicon may preferably have an average pore size (average pore diameter) of no more than about 200 nm, no more than about 100 nm, no more than about 50 nm, no more than about 25 nm, no more than about 20 nm, no more than about 15 nm, no more than about 10 nm, from about 1 nm to about 200 nm, from about 1 nm to about 100 nm, from about 1 nm to about 50 nm, from about 1 nm to about 20 nm, or from about 1 nm to about 10 nm, e.g. from about 5 nm to about 25 nm. The porous silicon may preferably have an average pore size of greater than about 1 nm, greater than about 2 nm, greater than about 3 nm, greater than about 4 nm or greater than about 5 nm. The pore size of the porous silicon starting material may be controlled by varying certain parameters in its synthesis, for example, by controlling the resistivity of the silicon, the concentration of hydrofluoric acid and oxidant in the etching solution and metal precursor concentration in metal assisted chemical etching and the voltage or current during anodic etching, as described in US 8,568,877 and US 5,358,600. Pore size may be measured using Barrett-Joyner-Halenda (BJH) analysis, as is known in the art.

The porous silicon starting material may itself be produced by etching silicon (e.g. crystalline silicon such as a silicon wafer). The silicon may be etched by any process known in the art. Examples of silicon etching processes include chemical etching, e.g. metal assisted chemical etching (MACE), and electrochemical etching (ECE). Silicon etching processes provide porous silicon particles comprising a continuous silicon skeleton with interconnected pores. Thus, the porous silicon particles used in the method of the present invention each preferably comprise a single continuous silicon skeleton. Each particle is thus preferably monolithic. Each particle is thus preferably not comprised of multiple smaller particles. This is a function of the process used to synthesise the particles, as described in further detail below. The porous silicon particles used as a starting material in the method of the present invention are thus fundamentally different in structure, properties and composition compared to particles produced by sintering or otherwise joining smaller silicon (or a silicon-containing material e.g. silicon monoxide) particles together.

Current methods to deliver bone regenerative agents rely on materials with macroscopic porosity with limited ability to control the release rate. For example, the delivery of BMP-2 and small molecule GSK3 inhibitors is typically done with collagen sponge. It would be expected that release from small pores (e.g. mesopores) would be instantaneous or very rapid. The particles made by the present method provide sustained release of the lithium, and othosilicic acid. However, without wishing to be bound by theory, it is thought that the present method advantageously results in the incorporation of lithium within the crystal structure of the porous silicon, following initial diffusion of lithium into the pores of the silicon starting material. The lithium can then be released from the particles in a sustained fashion in a biological environment with its release profile controlled by dissolution of the porous silicon skeleton/structure. The form of the product as (e.g. mesoporous) particles (e.g. micro or nanoparticles) make them ideal for incorporation into formulations, e.g. bone and dental cements, polymeric implants or injectable suspensions, for repair and regeneration of mineralised tissue, whilst unexpectedly providing sustained release of lithium.

Preferred properties and features of the lithiated porous silicon particles produced by the method of the present invention are as hereinbefore described in relation to the lithiated porous silicon particles per se. The method of the present invention advantageously enables the incorporation of higher levels of lithium than current lithium delivery technologies such as lithium-doped bioactive glass which suffer from poor loading degree and poor biodegradability of the bioactive glass, which limits the possibility to freely tune the lithium (and silicic acid) amount and release rate.

The method of the present invention comprises contacting porous silicon particles with a source of lithium. The porous silicon and source of lithium may be contacted together in the solid state, i.e. both the porous silicon and the lithium source may be in the form of a solid when contacted together. A preferred method of the present invention may therefore comprise contacting porous silicon particles with a source of lithium, wherein both the porous silicon particles and the source of lithium are in the solid state. This method is referred to hereinafter as the solid state method.

In a preferred solid state method, no solvent is used. In such a method the solid porous silicon particles and solid lithium source may be mixed together in the solid state (i.e. in the absence of any liquid or solvent), for example in a mortar or ball-mill. This method is advantageous as it is straightforward and avoids the use of extraneous solvents in the process.

Alternatively, the source of lithium (e.g. solid lithium source) may be dissolved in a solvent (e.g. a volatile solvent such as methanol) to form a solution, and contacted with the (solid) porous silicon, followed by removal of the solvent. Removal of the solvent thus leaves a mixture of porous silicon and the lithium source in the solid state. The porous silicon may be added to the solution of the lithium source in the solvent, or the solution may be added to the porous silicon. Removal of the solvent may be achieved by evaporation, such as by air drying, vacuum drying or drying in other gas, e.g. at room temperature or elevated temperatures. Removal of the solvent thus leaves the source of lithium and porous silicon in the solid state to react together to form the lithiated silicon product. Advantageously, this approach readily achieves intimate mixing of the lithium source and the porous silicon particles, enabling efficient insertion of the lithium into the porous silicon structure by utilising the large surface area of porous silicon. When a solvent is used, any suitable solvent in which the lithium source is soluble and which can be readily removed following mixing with the porous silicon may be used. Volatile solvents are preferred, e.g. a volatile organic solvent. The solvent may preferably be an alcohol, e.g. methanol, ethanol or i-propanol, preferably methanol. Alternatively the solvent may be aqueous such as water, or buffered solutions. Alternatively the solvent may be organic solvents such as DMSO, DMF, TFA, DCM.

The solid state method of the invention may be carried out at room temperature or at elevated temperature. In preferred solid state methods of the invention, the mixture of porous silicon and the source of lithium in the solid state is preferably heated to a temperature above the melting point of the source of lithium. The mixture of porous silicon and the source of lithium may preferably be heated to a temperature of from about 200 °C to about 1000 °C, more preferably from about 400 °C to about 900 °C. Heating may be carried out by any suitable method known in the art, for example using a hotplate, oven or furnace. Heating the mixture, particularly to above the melting point of the lithium source, facilitates the entry of the lithium source into the pores of the porous silicon particles, allowing greater penetration of the lithium into the silicon matrix. This may lead to benefits such as greater incorporation of lithium, providing a product with a higher lithium content, and deeper incorporation of lithium into the particles. This may lead to a more efficacious product and provide for sustained release of lithium from the product over a longer period of time.

The porous silicon and source of lithium may be contacted together for any length of time sufficient for incorporation of lithium into the porous silicon particles to occur. In the solid state method, the porous silicon and source of lithium may preferably be contacted together (e.g. heated together) for from 1 second to 24 hours, from 1 minute to 18 hours, from 10 minutes to 12 hours or from 20 minutes to 10 hours, e.g. from 30 minutes to 8 hours.

In the solid state method, any suitable lithium source may be used, for example those described above. Preferred lithium sources for the solid state method may include LiOH, LiCl and Li₂CO₃.

Depending on the temperature and the desired oxidation state of the product, the solid state method may be carried out in air or in a substantially non-oxidising (e.g. inert gas) atmosphere (e.g., nitrogen or argon), provided at least some oxygen is present, or any mixtures of these. Water vapour may be added to the mixture of porous silicon and lithium source. The addition of water vapour may accelerate oxidation of the product, which could be desirable because it controls the formation of lithium silicon oxides (lithium silicates) and regulates lithium incorporation as well as surface reactivity.

The methods of the invention produce lithiated porous silicon particles. The solid state method may further comprise washing the lithiated porous silicon particles. Washing the particles may advantageously remove excess salt components (e.g. originating from the source of lithium) whilst avoiding the degradation of the lithiated porous silicon and premature release of lithium. Washing may comprise dispersing the particles in a solvent, mixing the particles and solvent (e.g. by sonication or by grinding in mortar) and removing the solvent (e.g. by centrifugation). The washing steps may be repeated multiple times (e.g. 2, 3, 4, 5 or 6 times). An acidic solvent (e.g. aqueous HCl) or organic solvent (e.g. ethanol or isopropanol) may preferably be used for the washing, as this aids in avoiding degradation or dissolution of the lithiated porous silicon product.

As an alternative to the solid state method, a hydrothermal method may be used. Thus, in the method of the present invention the porous silicon and source of lithium may be mixed together in a liquid medium, preferably an aqueous medium. Preferably, the mixture in the liquid medium is heated, for example to a temperature of from 50 °C to 350 °C, preferably from 75 °C to 300 °C. Heating the mixture provides thermal energy which promotes effective diffusion of the lithium into the silicon matrix. Heating may be achieved, for example, using a thermomixer, water bath, oil bath sand bath or using a microwave reactor.

The mixture may preferably be heated to a temperature at or above the boiling point of the liquid medium, for example from about 100 °C to about 350 °C, or from about 150 °C to about 250 °C, e.g. from about 175 °C to about 200 °C. When the reaction mixture is heated to over, or close to, the boiling point of the liquid medium a closed (sealed) reaction vessel is preferably used, for example pressure resistant vials allowing lithiation to be carried out at high pressure and temperature. By using a microwave reactor, water vapour can increase the pressure can be increased, for example, 15 bars over ambient pressure reaching treatment temperatures of, for example, up to 180 °C.

In the hydrothermal method the mixture may preferably be heated under pressure, for example at a pressure of from 0 bar to 30 bar or from 5 bar to 20 bar, e.g. 15 bar over ambient pressure.

In the hydrothermal method, any suitable lithium source may be used, for example those described above. Preferred lithium sources for the hydrothermal method include lithium halides, e.g. LiCl.

In the method of the present invention wherein the porous silicon and source of lithium are mixed together in a liquid medium, preferably an aqueous medium (i.e. in the hydrothermal method), a pH tuning additive may be added to the reaction mixture. Preferred pH tuning additives include those which lower the pH of the mixture, for example silicic acid or HCl. The use of a pH tuning additive can advantageously minimise degradation of the silicon that may otherwise occur in an alkaline medium.

The porous silicon and source of lithium may be contacted together for any length of time sufficient for incorporation of lithium into the porous silicon particles to occur. In the hydrothermal method, the porous silicon and source of lithium may preferably be contacted together (e.g. heated together) for from 1 minutes to 48 hours.

The present invention also provides lithiated porous silicon particles formed by the method hereinbefore described.

### Examples

### Example 1 - Synthesis of porous silicon nanoparticles

Fabrication processes to generate porous silicon are known. Two alternative processes are metal assisted chemical etching (MACE) and electrochemical etching (ECE).

In metal assisted chemical etching (MACE), a silicon wafer is first covered with a noble metal layer. Silver, Gold, Palladium, Platinum and Copper have all been successfully used, each having different parameters for optimal etching. The metal layer can be deposited as a uniform layer by evaporation or sputtering or as a dense nanoparticle field by electroless deposition from metal salts in an aqueous solution of hydrofluoric acid (HF). The metal layer can be patterned by lithography, to limit the region of metal deposition and generate nanostructures/nanoparticles with desired geometry.

The process used herein typically employs a dense field of silver nanoparticles deposited from AgNO₃ solution. Following metal deposition, the wafer is covered in a solution containing HF and hydrogen peroxide (H₂O₂) as oxidant (nitric acid (HNO₃) may alternatively be used). The metal particles catalyze hole injection into silicon which causes local oxidation and dissolution of silicon due to the presence of HF, forming silicon nanowires/nanoplatelets. Local diffusion of the holes induces porosification of the non-etched silicon in the neighbourhood of the particle, controlling the porosity of the nanowires formed. Initial photolithography controls the shape of the base of the silicon nanostructure enabling forming particles with tunable aspect ratio. The porosity of formed nanowires can be controlled with process time and H₂O₂ concentration. After etching, the catalysing metal nanoparticles are dissolved using standard metal etchant solution, wafer is cleaned and porous silicon nanowires are detached with a sharp blade.

More details on the MACE process can be found in Chiappini et al, adv func mater 2010 and in the patent US8568877B2 - POROUS AND NON-POROUS NANOSTRUCTURES

In electrochemical etching (ECE), an electrochemical cell is formed with a silicon wafer acting as the anode facing a platinum cathode with an electrolyte containing HF and water. Typically, the solution also contains ethanol to control the HF concentration and provide wettability to the porous silicon structure as it is formed. Applied current through the cell causes the hole diffusion towards the interface of silicon and electrolyte, weakening of Si-Si bonds on the surface and inducing subsequent local dissolution of silicon leading to pore formation. The current density, type of used silicon, lightning condition and solution composition determine pore size and porosity of the porous silicon structure. This process yields continuous porous silicon layers. Altering the current during the process generates multiple layers with tunable porosity. After porous layer (or multilayer) is etched, high current density can be used to generate a fracture between the porous silicon and the underlying silicon wafer allowing lift-off of the entire the porous (multi)layer.

It is also possible to make porous silicon microparticles and nanoparticles with controlled geometry by first patterning the substrate and then performing ECE, for example as described in WO2008134637.

Nanoparticles are generated from ECE and MACE porous silicon by fracturing. Possible methods include ball milling, microfluidic processes, and ultrasonication. During the ultrasonication the particles are dispersed into a liquid medium and are fractured using a probe or tip sonicator, or a sonic bath, for the desired duration. Size selection is achieved by filtering or centrifugation. Alternatively, particles can be fractured and size selected after the lithiation.

In the examples set out below, the porous silicon particles starting materials were made using the following MACE process, unless otherwise stated:

### Facilities

Fabrication of porous silicon involves use of hydrofluoric acid (HF) which can lead to severe health issues if operator is exposed. Good training, specific chemical hood with appropriate PPE are needed.

### Materials for MACE

- Polished silicon prime wafer: diam 100mm, dopant boron, resistivity 0.01-0.02 Ohm.cm, orientation (100), thickness 525 µm,
- Cleaning solution: 20ml 49% HF + 80ml DI H₂O
- Deposition solution: 5ml of aqueous 0.4M AgNO₃ + 20ml 49% HF + 75ml DI H₂O
- Etching solution: 6ml 35% H₂O₂ + 80ml 49% HF + 314ml H₂O
- Potassium iodide gold etchant

### Protocol

0) All steps are done in PTFE or PE dishes in NTP conditions.
1) Silicon wafer is incubated 2 minutes in cleaning solution.
2) Cleaned wafer is incubated 2 minutes in deposition solution while gently stirring the vial.
3) Coated wafer is rinsed sequentially in water, isopropanol and dried in dry air/nitrogen flow.
4) Wafer is incubated 20 minutes in etching solution.
5) Etched wafer is rinsed sequentially in water, isopropanol and dried in dry air/nitrogen flow. Etched wafer is incubated 10 minutes in gold etchant solution.
6) Wafer is rinsed sequentially in water, isopropanol, acetone and dried in dry air/nitrogen flow.
7) Nanowires are scraped from the wafer with sharp blade. Isopropanol can be used to avoid dusting.

### Weighting the particles

1) If particles are dry, weight is directly measured with balance by using non-static sample container.
2) If particles are in suspension, 0.5ml sample is withdrawn to the vial with known weight, solvent is let to evaporate, vial and particles are weighted with the balance and mass of nanoparticles is calculated.

### Example 2 - Lithiation of porous silicon particles

### Solid state (SS) method

In this method, the porous silicon is directly mixed with lithium containing agents, typically solid lithium salts and the mixture is heated. In this process, the critical parameters are type of lithium source, the mixing method, the treatment temperature(s), time and atmosphere, and the washing protocol after the treatment. Lithium salts such as LiCl, LiOH, LiOH*H₂O, Li₂CO₃, lithium citrate, and others can be used. Mixing can be done in mortar or ball-mill, for example and addition of lithium salt dissolved in volatile medium such as methanol can be used. In the latter case, porous silicon is mixed with this solution and the solvent is evaporated before the thermal treatment. Thermal treatment can be done in hotplate, oven or furnace and the temperature ranges from room temperature up to 1000 degrees. Depending on the temperature and desired oxidation state, the treatment can be done in air or inert gas atmosphere (such as N₂ or argon), or mixture of these, provided at least some oxygen is present. Also, water vapor can be added to accelerate oxidation. Thorough washing following treatment removes excess salt components while avoiding the degradation of porous silicon and premature release of lithium at the same time. Washing is done by dispersing particles in different solvents, grinding in mortar, sonicating to mix and centrifuging to remove the solvent. Acidic or organic washing solvent can be used to avoid degradation or dissolution of the product. Multiple cycles of washing and spinning down may be advantageous.

Particle size reduction and size separation can also be performed after washing. Lithiated feedstock particles can be ball-milled, milled, grinded, ultrasonicated and after that the desired size fraction can be separated with centrifuge or filter. Particle size reduction and separation may also/alternatively be carried out on the original pSi particles prior to lithiation.

### Hydrothermal (HT) method

In this method porous silicon is immersed into a liquid (typically aqueous) medium containing the lithium source. Lithium can be added as a salt such as LiCl. Effective lithium diffusion into silicon matrix requires additional energy, which can be provided by heating the reaction mixture with thermomixer, water bath, oil bath, sand bath or using a microwave reactor. Heating over, or close to, the boiling point of the liquid increases the pressure within the closed reaction vessel. In this case, special pressure resistant vials are used allowing performing lithiation at high pressure and temperature. In the microwave reactor, water vapor pressure can be increased 15 bars over ambient pressure reaching treatment temperatures up to 180°C. In order to avoid/reduce/control degradation of silicon in alkaline medium, pH tuning additives such as silicic acid or HCl can be used.

### Reaction conditions

Various lithiated porous silicon (LipSi) particles were synthesised using either the solid state or hydrothermal method described above. The reaction conditions used are set out in Table 1 below. For SS16 the ECE method was used to from the porous silicon particle starting material. For all other samples the MACE method was used. The amount of pSi particles used varied from 5mg to 30mg. Solid state reactions were either carried out by mixing solid pSi and solid Li source in a mortar or by dissolving the Li source in methanol, mixing with pSi then evaporating the methanol off.

**Table 1**

| **Sample** | **Method** | **Lithium source** | **Si:Li molar ratio** | **Temp** | **Time (hours)** | **Mixing method** | **Medium** |
|---|---|---|---|---|---|---|---|
| SS8 | Solid state | LiOH | 1:1 | 200+800°C | 0.5 | Mortar | Air |
| SS9 | Solid state | LiOH | 2:3 | 450 °C | 0.5 | Mortar | Air |
| SS10 | Solid state | LiOH | 1:1 | 200+450°C | 24+0.5 | Mortar | Air |
| SS11 | Solid state | LiOH | 2:3 | 450 °C | 0.5 | Mortar | Air |
| SS12 | Solid state | LiOH | 2:3 | 450 °C | 0.5 | Mortar | Air |
| SS13 | Solid state | LiOH | 1:1 | 200+450°C | 24+0.5 | Mortar | Air |
| SS14 | Solid state | LiOH | 2:3 | 800°C | 0.5 | Mortar | Air |
| SS16 | Solid state | LiOH | 2:3 | 450 °C | 7 | Mortar | N₂ |
| SS17 | Solid state | LiOH | 2:3 | 450 °C | 7 | Mortar | Air |
| SS19 | Solid state | LiCl | 3:7 | 450 °C | 4 | Evaporation | Air |
| SS20 | Solid state | Li₂CO₃ | 3:7 | 450 °C | 6 | Mortar | N₂ |
| SS21 | Solid state | LiCl | 3:7 | 450 °C | 5 | Evaporation | N₂ |
| SS32 | Solid state | LiCl | 3:7 | 800 °C | 0.5 | Evaporation | Air |
| SS33 | Solid state | LiCl | 3:7 | 650 °C | 0.5 | Evaporation | Air |
| HT90 | Hydrothermal | LiCl | 1:85 | 90 °C | 24 | Evaporation | Aqueous |
| HT180 | Hydrothermal | LiCl | 1:141 | 180 °C | 12 | Evaporation | Aqueous |

HT180 was prepared using a microwave reactor. The equilibration pressure varied from 10 to 14bars. HT90 was prepared in a closed container in a water bath.

### Example 3 - Lithium content of lithiated porous silicon particles

The absolute amount of lithium and silicon in the synthesised particles was measured by dissolving 100 µg/ml LipSi particles into 1M KOH. Solutions were incubated 20-72 days in and shaken regularly until all the particles were dissolved. Experiment was repeated 2-3 times and averages are reported. Dissolved samples were diluted 20 times into 1% HNO₃ and elemental composition was measured with Inductively Coupled Plasma Mass Spectrometry (ICP-MS).

The results of the analysis are shown in Table 2 below.

**Table 2**

| | **Li/Si (%)** | | |
|---|---|---|---|
| **Sample** | **Average** | **Standard deviation** | **N** |
| SS8 | 4.09 | 0.95 | 3 |
| SS9 | 2.00 | - | 1 |
| SS10 | 0.93 | 0.17 | 3 |
| SS11 | 0.98 | 0.08 | 3 |
| SS12 | 1.07 | 0.11 | 3 |
| SS13 | 0.54 | 0.07 | 3 |
| SS14 | 0.82 | 0.08 | 3 |
| SS16 | 0.30 | 0.03 | 3 |
| SS17 | 0.28 | 0.01 | 3 |
| SS19 | 5.20 | 0.26 | 2 |
| SS20 | 0.35 | 0.04 | 2 |
| SS21 | 1.39 | 0.05 | 2 |
| SS32 | 73.93 | - | 1 |
| SS33 | 42.35 | - | 1 |

The results show that the amount of lithium (relative to silicon) in the samples could be controlled from 0.1 to 73.93 wt%. The temperature and method of lithiation allows control over the amount of lithium incorporated.

SS32 and SS33 demonstrate that a high Li/Si content can be achieved. In the case of SS32 the detected Si concentration is 8.8 ug/ml while the detected Li concentration is 6.51 ug/ml (giving 74% Li/Si), but the starting amount of material was at 100 ug/ml concentration, so the remaining 84.69% of material is not captured in the ICP-MS analysis and at least some of this will be oxygen. In the case of SS33, 7.91 ug/ml of lithium was detected in 100 ug/ml particles (giving 42% Li/Si).

### Example 4 - Lithium and silicon release

The release of lithium and silicon from the particles was studied by incubating the LipSi particles in physiological pH 7.4 phosphate buffer at 37 degrees. Concentration of the particles was kept below solubility saturation limit of 100 µg/ml. Concentration of released ions was measured with ICP-MS. A sample of non-lithiated porous silicon particles was used as a control.

The results are shown in Figure 1, which shows the amount of released lithium or silicon in µg over time. The results show that LipSi particles showed sustained released of lithium and silicon compared to free LiCl and pSi particles.

Lithium release is tailorable and can be sustained between 1 and 14 days and it releases in a similar fashion to silicon. Eventually lithium release slows down to approximately zero while silicon still gets released at an approximately constant rate up to 21 days, suggesting that lithium is incorporated up to a definite thickness from the surface of the particles (including the outer particle surface and the inner surfaces of the pores).

### Example 5 - Structural characterisation

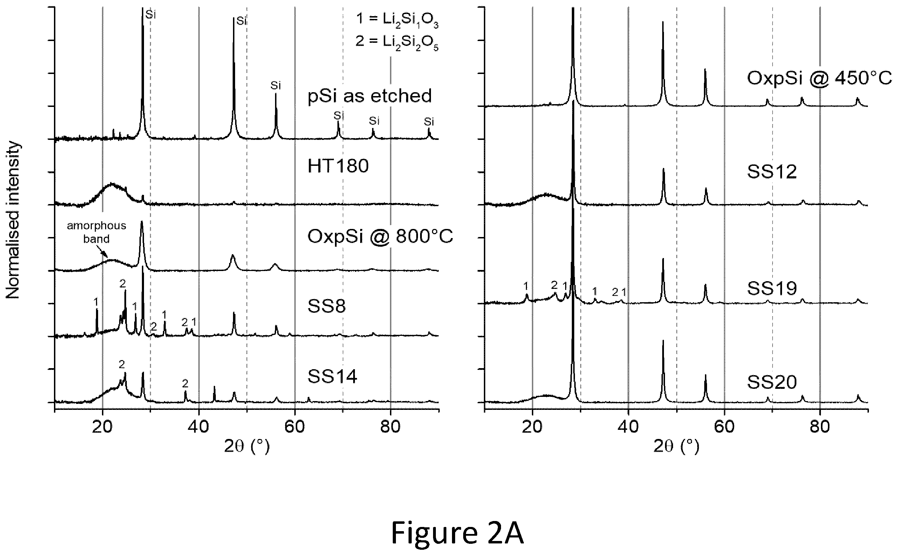

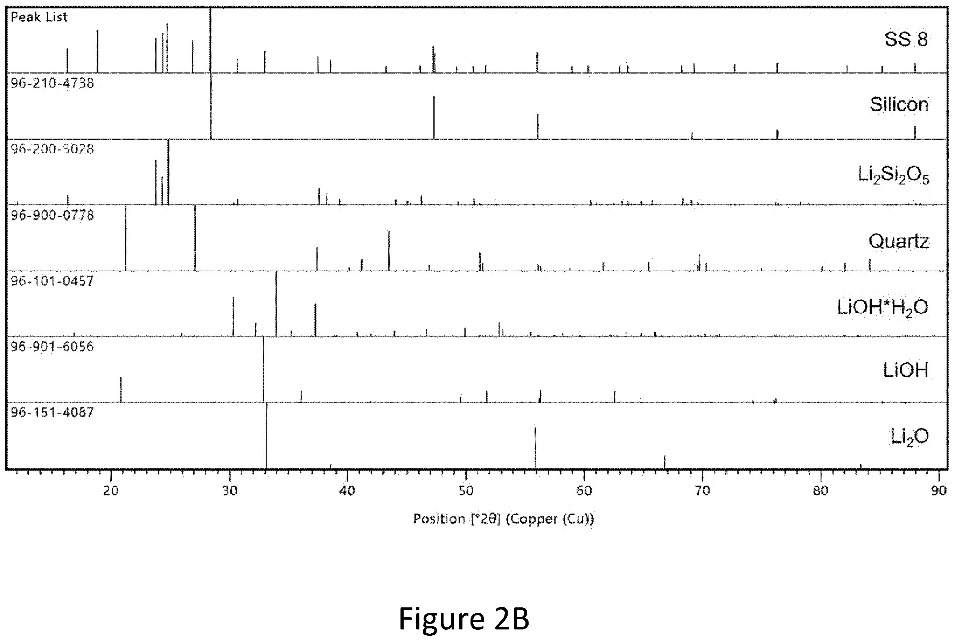

An X-Ray Diffraction (XRD) study of the LipSi particles was done in order to understand more about how the lithium is incorporated within the silicon matrix. The XRD diffractograms are shown in Figure 2A. XRD peaks were assigned by comparison to reference materials comprising lithium and silicon, as shown in Figure 2B. The various species identified in the samples are outlined in Table 3 below.

**Table 3**

| **Sample** | **XRD** | | | |
|---|---|---|---|---|
| | **Si** | **Li₂SiO₃** | **Li₂Si₂O₅** | **amorphous** |
| HT180 | - | - | - | x |
| SS8 | x | x | x | x |
| SS12 | x | - | - | x |
| SS14 | x | - | x | x |
| SS19 | x | x | x | x |
| SS21 | x | - | x | x |
| OxpSi @ 800°C | x | - | - | x |
| OxpSi @ 450°C | x | - | - | - |
| pSi as etched | x | - | - | - |

The results show that all LipSi particles made by the solid state method were found to have clear peaks of crystalline silicon (Si) and almost all of them had an amorphous fraction.

Porous silicon oxidized in air at 450°C for 30 minutes (OxpSi @ 450 °C) does not show amorphous peaks, as the particles are made from single crystal silicon and the temperature is insufficient for oxidation into an amorphous SiOₓ phase.

Instead, lithiation at 450°C shows an amorphous peak that can be attributed to amorphous LiₓSi species due to interstitial lithium.

800°C oxidization of porous silicon (OxpSi @ 800 °C) shows an amorphous peak from SiOₓ phase.

For 800°C treatment of LipSi direct evidence of formation of crystalline lithium meta- and disilicates during the lithiation was found (Li₂SiO₃ and Li₂Si₂O₅, respectively).

For lower temperature treatments, these silicates can also be found. When lithiation was performed with LiCl in a Si:Li ratio 3:7 the appearance of lithium silicates was reproducible. Crystalline phases of SiO₂, LiOH, LiOH·H₂O, LiCl, LiCl·H₂O, Li₂O, Li₂₂Si₅, Li₁₃Si₄, Li₇Si₃ and Li₁₂Si₇ were not observed in samples.

### Example 6 - MAS-NMR

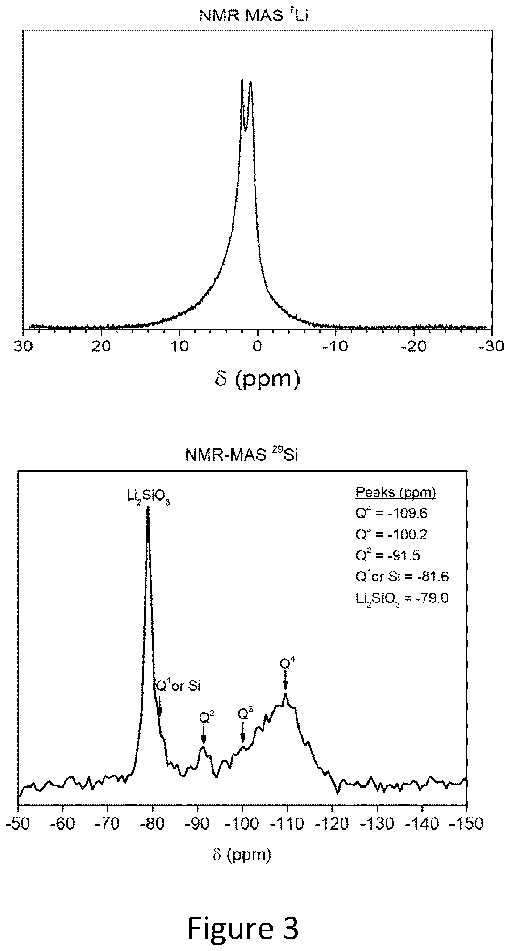

MAS-NRM ⁷Li and ²⁹Si NMR spectra were measured on a 400 MHz NMR spectrometer with Bruker Avance console operating at frequency of 194.35 MHz. The results for sample SS21 are shown in Figure 3.

The results of ⁷Li measurement (Figure 3; left hand spectrum) shows the presence of multiple lithium species, including silicides and peak broadening compared to LiCl indicating to the change of chemical environment of Li-ions. The results of ²⁹Si measurement (Fig 3; right hand spectrum) shows also presence of multiple silicon species and Li₂SiO₂. Peak broadening are attributed to amorphicity.

### Example 7 - TEM data

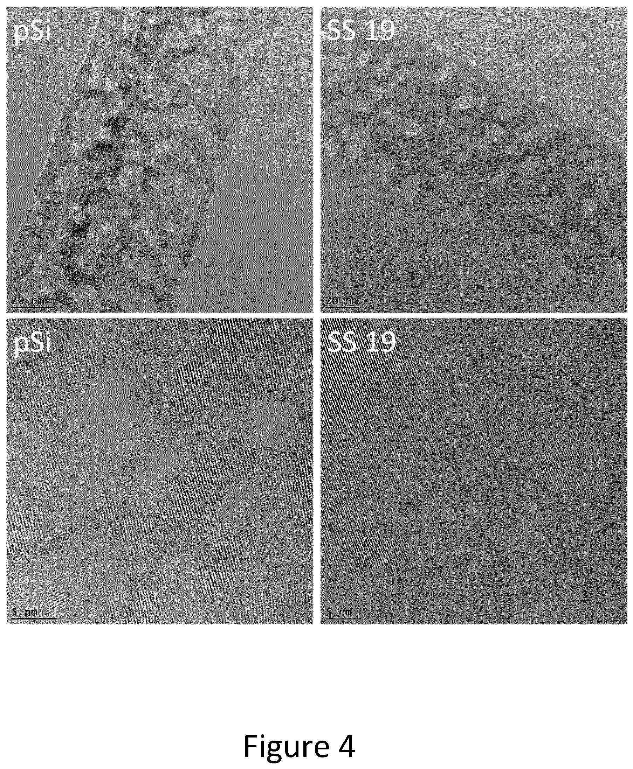

The structure of the LipSi particles was measured using high-resolution transmission electron microscopy (HRTEM). The results are shown in Figure 4.

The results confirm the crystallinity of LipSi sample SS19 and reference pSi, as observed also in XRD. The images also show that LipSi nanoparticles, when compared to porous silicon, maintain their shape and porosity.

### Example 8 - XPS

The chemical composition of the LipSi particle surface after lithiation was measured with X-ray photoelectron spectroscopy (XPS). XPS Analysis was performed using a Kratos Axis SUPRA XPS fitted with a monochromated Al ka X-ray source (1486.7 eV) equipped with an Ar cluster gun for sputtering.

The results (in atomic %) are shown below in Tables 4A and 4B. The pre-etch XPS data is representative of the composition of the particle surface. The post-etch data looks at the XPS signature after the particles have been sputtered so the surface has been removed. Since the material is highly porous the post-etch is still a mixture of surface and volume and thus is more representative of the overall particle composition.

**Table 4A**

| | **XPS (at%)** | | | | | | | |
|---|---|---|---|---|---|---|---|---|
| | **Before argon etching** | | | | **After argon etching** | | | |
| | **Siₒₓ/Siₜₒₜ** | **Si₀/Siₜₒₜ** | **Li/Siₜₒₜ** | **Li/Siₜₒₜ** | **Siₒₓ/Siₜₒₜ** | **Si₀/Siₜₒₜ** | **Li/Siₜₒₜ** | **Li/Siₜₒₜ** |
| **Sample** | **at%** | **at%** | **at%** | **m%** | **at%** | **at%** | **at%** | **m%** |
| SS8 | 94.3 | 5.7 | 37.8 | 9.3 | 93.3 | 6.7 | 3.6 | 0.88 |
| SS9 | 86.7 | 13.3 | 39.8 | 9.8 | 81.2 | 18.8 | 2.7 | 0.67 |
| SS12 | 92.7 | 7.3 | 38.7 | 9.5 | 86.6 | 13.4 | 3.6 | 0.88 |
| SS13 | 89.6 | 10.4 | 0.0 | 0.0 | 90.0 | 10.0 | 0.0 | 0.0 |
| HT180 | 98.6 | 1.4 | 18.5 | 4.6 | 99.8 | 0.2 | 5.8 | 1.42 |
| pSi | 35.8 | 64.2 | 0.0 | 0.0 | 32.4 | 67.6 | 0.0 | 0.0 |

**Table 4B**

| | **Pre-etching** | | | | | | | **Post-etching** | | | | | | |
|---|---|---|---|---|---|---|---|---|---|---|---|---|---|---|
| **Peak** | **HT180** | **HT90** | **pSi** | **SS13** | **SS12** | **SS9** | **SS8** | **HT180** | **HT90** | **pSi** | **SS13** | **SS12** | **SS9** | **SS8** |
| O 1s | 94.5 | 94.8 | 87.3 | 93.7 | 93.7 | 83.2 | 92.7 | 66.8 | 63.9 | 44.9 | 65.4 | 56.4 | 49.2 | 60.2 |
| Li 1s | 0.87 | 0.00 | 0.00 | 0.13 | 1.73 | 8.44 | 1.97 | 3.21 | 1.11 | 0.00 | 0.37 | 1.98 | 3.21 | 1.31 |
| Si 2p | 4.6 | 5.2 | 12.7 | 6.2 | 4.5 | 8.4 | 5.3 | 30.0 | 35.0 | 55.1 | 34.2 | 41.6 | 47.6 | 38.5 |

The results show that all lithiation approaches induce oxide (can be any SiOx species including lithium silicates) formation at the surface silicon.

The lithium content drops markedly following Argon sputtering (see release studies above) indicating that the lithium is introduced up to a definite thickness from the surface of the pores.

### Example 9 - Raman Spectroscopy

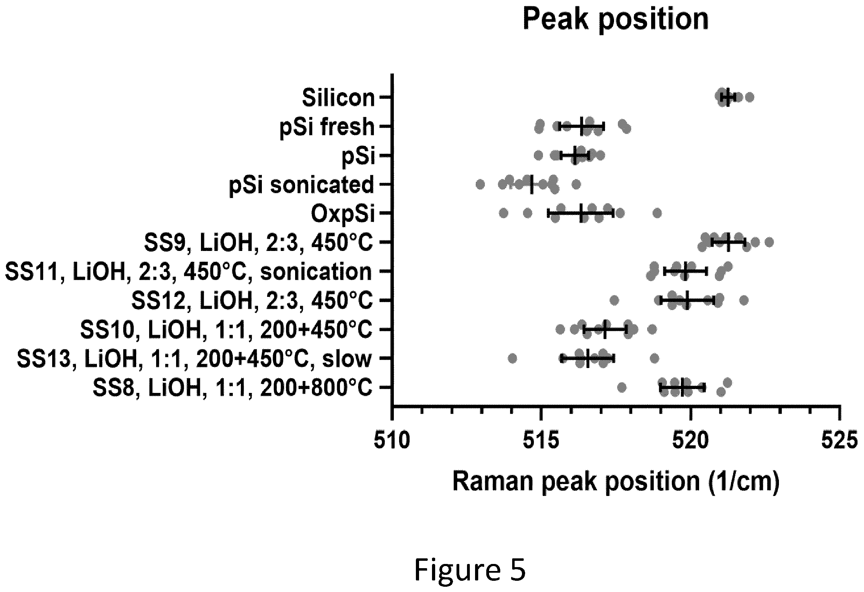

Structural alterations and strains in the silicon crystal lattice may cause also a shift in silicon Raman peak (at 520 1/cm), which provides indirect evidence of Si-Li interaction. Figure 5 shows the position of the silicon Raman peak for various LipSi samples.

The results show that etching of silicon and subsequent creation of porous silicon, caused a red shift in the silicon peak, which is well known in the literature. Oxidation of porous silicon does not affect the peak position.

Some lithiation processes induce a blue shift, suggesting interstitial insertion within the crystal lattice of silicon. In LipSi samples SS8, SS9, SS11, SS12, the shift was observed. In samples SS10 and SS13, the shift was not observed. Interestingly, the shift of the Raman peak correlates with the overall lithium content in particles measured with ICP-MS.

### Example 10 - Co-localization of Li and Si signals

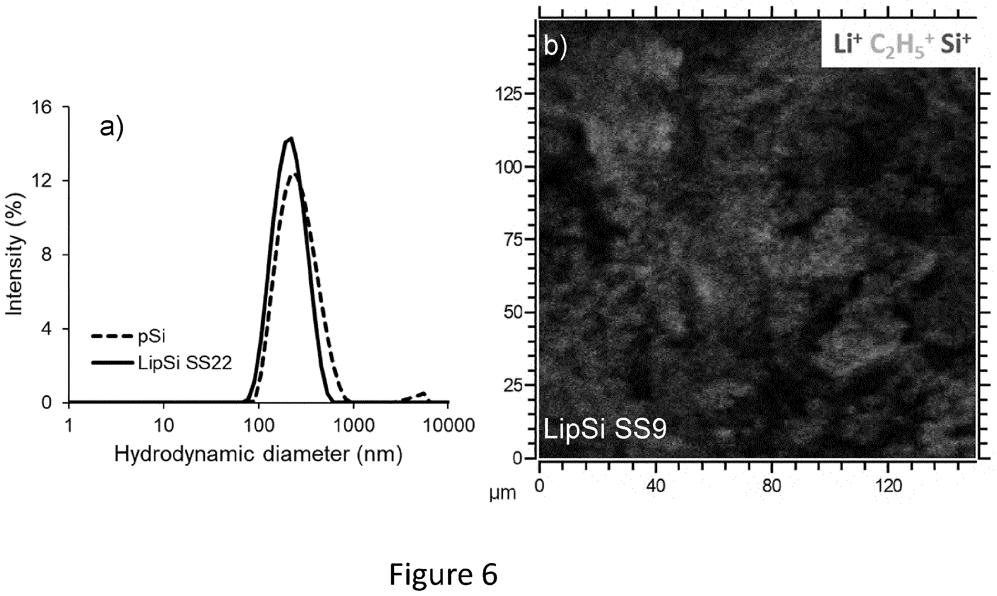

Co-localisation is studied with secondary-ion mass spectrometry (SIMS) measurements (Fig 6). LipSi particles (SS9) were dried onto carbon substrate and the amount of lithium and silicon was probed with SIMS yielding to spatial distribution image where different colours represent different ions. The measurement shows overlapping of lithium and silicon signal indicating that the lithium is incorporated into silicon particles and not present without silicon.

### Example 11 - Bioactivity in vitro preliminary study

Biological activity of released lithium was measured by monitoring activation of Wnt/β-catenin cell signalling pathway by measuring the expression of the pathway's well-known downstream target gene Axin2. The experiment was done with cell medium conditioned with corresponding batch of nanoparticles. Known amounts of LipSi nanoparticles were pelleted with a centrifuge and sterile supernatant was removed. Nanoparticles were dried under tissue culture hood. Cell medium (αMEM supplemented with 10% FBS, 1% antibiotic / antimycotic solution and 25mM HEPES) was added onto pellet and particles were redispersed with ultrasound. Concentrations of the particles were 0.8 mg/ml (Figure 6a) and 1.6 mg/ml (Figure 6b). Vials were incubated in thermoshaker at 37°C for four days. Particles were centrifuged down and supernatant was collected under sterile conditions. Conditioned medium (supernatant) was filtered with 0.22µm syringe filter.

20,000 cells/cm² of mesenchymal cells (17IA4) were seeded onto 24 well plate. Cells were incubated approximately for 24 hours until 80% confluent and the normal medium was changed to conditioned. Cells were incubated (37°C, 5% CO₂) in conditioned medium for 24 h. Cell medium was removed, cells were lysed and RNA was extracted with RNeasy Mini Kit (Qiagen cat no. 74104) according to product's protocol. qPCR for Axin2 expression was performed on the cell lysate. Reported fold changes represent Axin2 expression in the sample compared to Axin2 expression in medium and they are calculated with ΔΔCₜ method as known in the art.

The results are shown in Figure 7a) and b). Significant, 2.2- and 1.9-fold, increase of Axin2 expression was found with LipSi samples SS9 and SS19 (Figure 6a, Figure 6b) with the one-way Anova test p value <0.01 and <0.001, respectively. Treatment of the cells with 25 mM LiCl increased the Axin2 expression 3.2- and 2.6-fold with p value <0.001 and <0.0001.

In both cases the sample with highest lithium content, 2.0% for SS9 and 5.2% for SS19, caused the increased expression of Axin2 which indicates that the increase is due to the released lithium.

Viability of cells, exposed 1 day to LipSi and pSi particles was tested with ATP assay. The viability assay was done with suspension of particles and cell medium prepared as before excluding 4d conditioning, centrifuging and filtering. Mesenchymal cells (17IA4) were seeded as before and cell medium was changed to the medium with nanoparticles when cells were 80% confluent. After 24h cell viability was tested with CellTiter-Glo Luminescent Cell Viability Assay (Promega, cat.no. G7570) according to product's protocol. Reported viability represents the measured viability signal from cells incubated with nanoparticles compared to the cells incubated with medium only. Surfactant Triton X-100 is used as cytotoxic positive control.

Lithiated pSi particles (SS11 and HT90) showed non-significant (ns) decrease of viability at all concentrations between 50 and 800µg/ml when incubated with dental pulp mesenchymal like cells 17IA4 (Figure 7c). Native (as etched) pSi particles instead caused a significant (p<0.0001) decrease of viability at concentrations above 100µg/ml. The cytotoxicity of native pSi particles is known in the art and typically attributed to the chemical instability and hydrophobicity of native silicon surface. Lithiation passivates the surface as also seen in release experiments and makes it hydrophilic thus increasing the particles' cytocompatibility.

### Example 12 - Bioactivity in vivo preliminary study

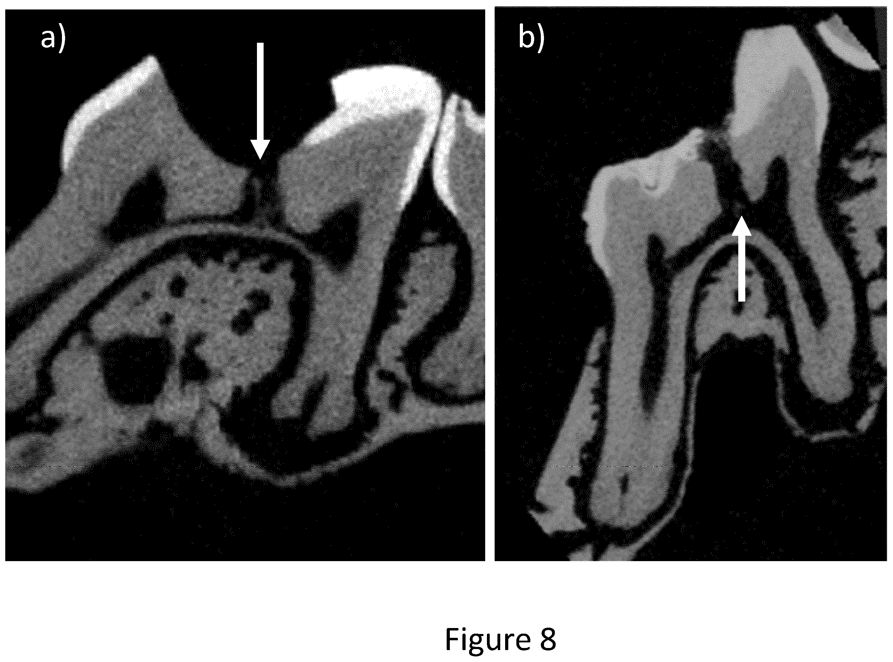

A preliminary study of particles (combination of similar batches SS11 and SS12) bioactivity was done with an *in vivo* dental model. A rounded carbide burr FG 1/4 was used to create cavity into mouse superior molar teeth. Once the burr cut exposed the dentine, a 30G needle was used to penetrate the pulp. LipSi nanoparticles in PBS were added directly to the exposed pulp and a composite of pSi and polyacrylic acid (PAA) was used to seal the pulp. Traditional glass ionomer capping was made after the composite was setted. After 4 weeks, mice were sacrificed, upper molars were dissected and fixed with PFA 4% overnight and scanned with Bruker Skyscan1272 micro-CT. As a control, only PBS and negligible amount of DMSO was injected and mice were sacrificed after 6 weeks. The images of a LipSi treated mouse molar in sagittal direction is shown in Figure 8a. Image of non-treated sham surgery control in sagittal direction is shown in Figure 8b.

The results show more formation of mineralised tissue (white in images) in the exposed pulp cavity (see the arrows) in LipSi treated particles after 4 weeks compared to spontaneous regeneration after 6 weeks.

### Example 13 - Lithium and silicon localisation with TEM/EELS

Lithiated porous silicon particles were dispersed from solution on a transmission electron microscopy (TEM) grid. The data was collected on an FEI Titan3 Themis G2 operated at an accelerating voltage of 300 kV, equipped with a field emission gun (X-FEG) operating at an extraction voltage of 4.5 kV and a monochromator. High-resolution (HR) TEM images and electron diffraction patterns were collected on a Gatan OneView CMOS camera.

Scanning transmission electron microscopy-electron energy loss spectroscopy (STEM-EELS) data was collected using a Gatan Quantum 965 ER energy filter for dual EELS (allowing for core-loss edges to be calibrated against simultaneously acquired low-loss spectra). A 0.1 eV/channel dispersion and 5mm entrance aperture were used. Collected spectra were processed in Gatan Microscopy Suite by applying a power law background subtraction.

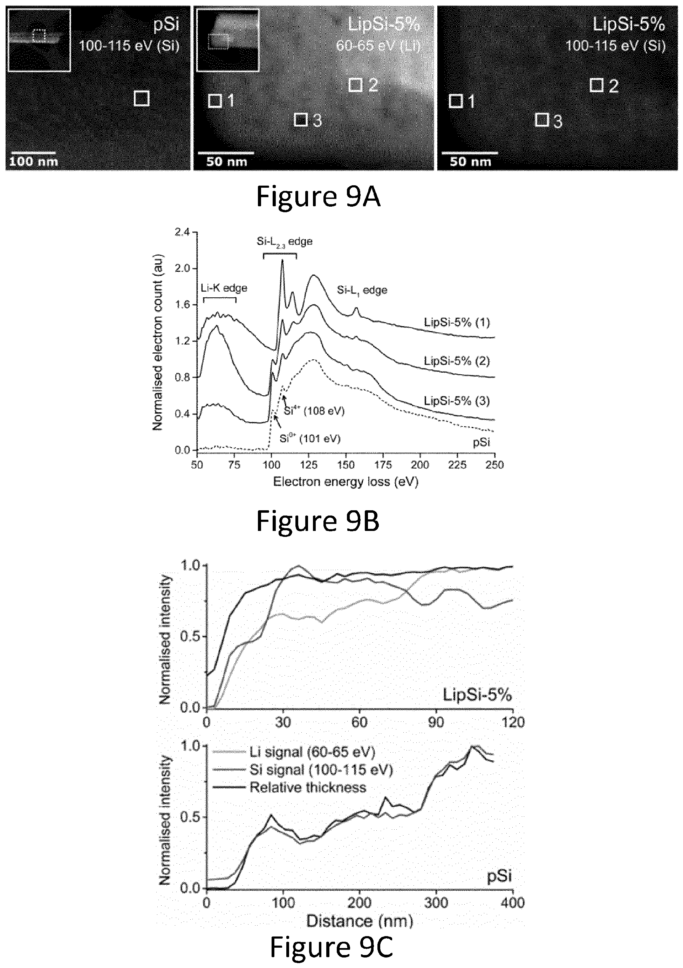

The results are shown in Figure 9. Figure 9A shows the map of relative concentration of silicon and lithium within a porous silicon and a lithiated porous silicon particle. The porous silicon particle does not contain lithium, while the lithiated porous silicon particles shows the co-localised presence of silicon and lithium within its skeleton. Figure 9B shows the electron energy loss spectra at the lithium and silicon absorption edges. The spectrum for the porous silicon particle (dotted line) does not show any presence of lithium, while the spectra from the three regions of the lithiated porous silicon particle show the presence of both silicon and lithium and the chemical interaction between the two elements in the fine structure of the silicon edge. Figure 9C shows the relative silicon and lithium concentration as well as the particle thickness along a line through the porous silicon and lithiated porous silicon particles. The lithiated porous silicon particle (upper graph) shows a higher relative abundance of lithium to silicon closer to the particle surface, while the porous silicon particle (lower graph) does not show any presence of lithium.

The results show that the lithiated porous silicon particles displayed the presence of silicon and lithium throughout the particles. Two different regions were detected. Close to the surface of the particles a higher proportion of lithium to silicon was observed, while in the particle interior the amount of silicon increased. The fine structure of the Si-L2.3 and Si-L1 EELS edges displayed a different profile close to the surface than in the bulk, indicating different chemical bonding for the Si in the bulk and close to the surface, due to the differing lithium slicates and lithium silicides species present throughout the particle.

### Example 14 - Pore size and porosity

Porosity characterization of the materials was done with N₂ sorption at 77K using TriStar 3000 (Micromeritics Inc.). Surface area determinations were also done using Kr sorption at 77K using 3Flex 3500 (Micromeritics Inc.). Specific surface areas were calculated using the BET method. Pore structure was estimated with density functional theory calculations from the N₂ sorption results using a silica-based model for cylindrical pores (MicroActive v.5.01, Micromeritics Inc.).

Results are set out in Table 5 below. Entries 1 and 2 relate to porous silicon particles with no lithium incorporated. Entries 3-5 relate to porous silicon particles lithiated using the solid state method under the stated lithiation conditions.

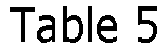

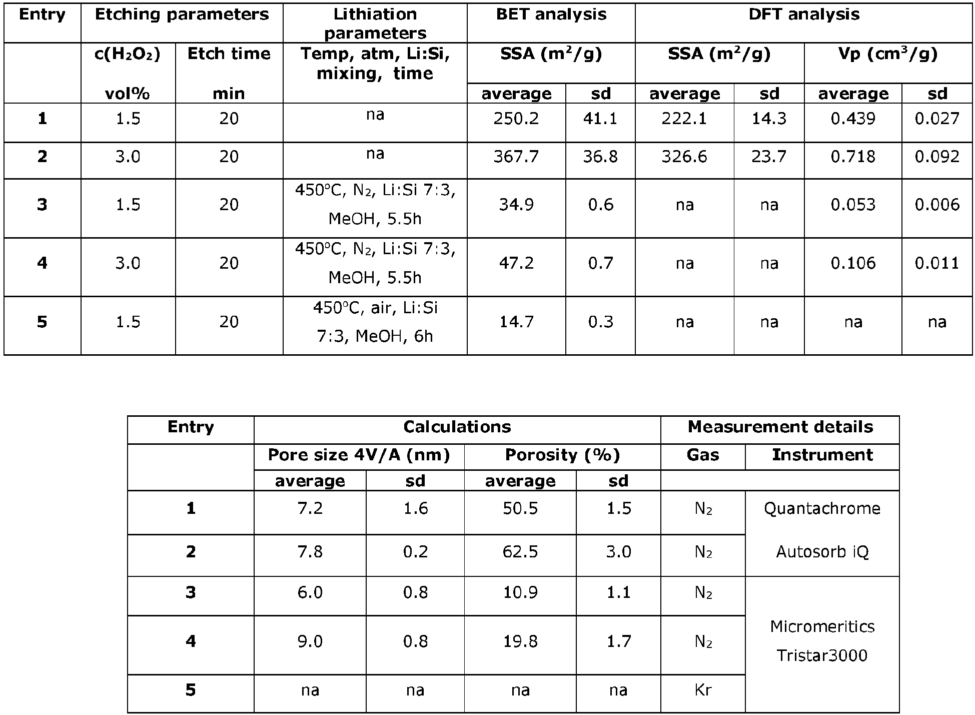

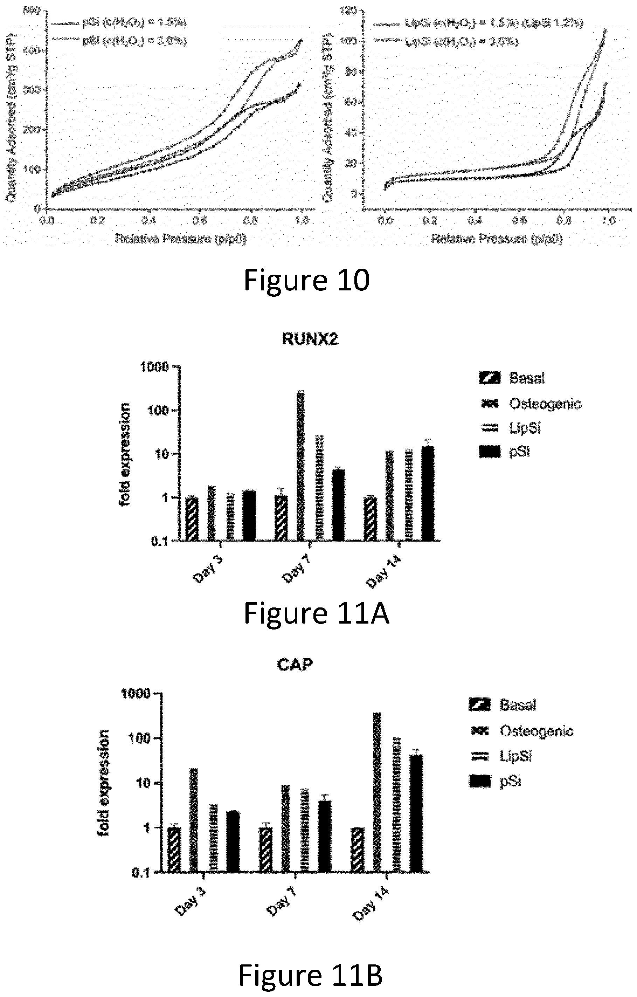

The nitrogen sorption curves (Figure 10) indicate that both porous silicon and lithiated porous silicon particles exhibit the characteristic mesoporous structure of porous silicon. By controlling the porous silicon etching conditions, for example by varying oxidant concentration in the MACE etching solution, it is possible to control porosity and pore size for the porous silicon particles. The lithiation process retains similar pore size to the original porous silicon particles, while also retaining particle porosity. Porous silicon particles with higher porosity retain higher porosity upon lithiation in similar conditions. The insertion of the lithium within the silicon expands the crystal lattice which in turn makes the solid region occupy more space, resulting in lower porosity in the LipSi particles compared to the pSi particle starting material. However, the fact that the LipSi particles are still porous is important as they retain the advantageous properties, such as solubility and the ability to carry/load a payload, of the porous silicon starting material.

### Example 15 - Bioactivity in vitro

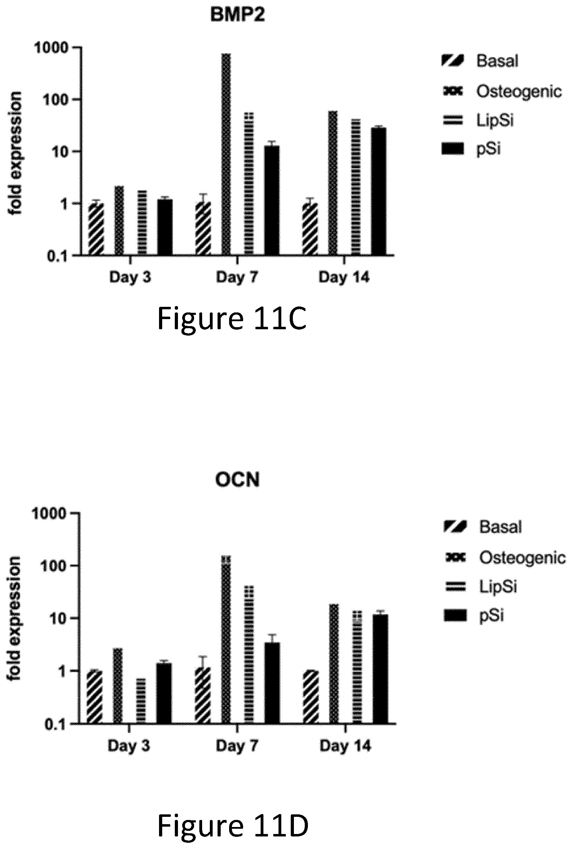

Primary human periodontal stem cells were exposed to different medium compositions to evaluate the activation of osteogenic (BMP2, RUNX2, OCN) and cementogenic (CAP) genes. The media evaluated were basal maintenance medium, osteogenic medium, basal medium conditioned with LipSi (SS21) and basal medium conditioned with pSi. The conditioning process involved incubating the medium for 4 days at 37 °C in the presence of the particles. At predefined timepoints (3 days, 7 days and 14 days) the cells were harvested and RNA extracted for analysis by qPCR. The results are shown in Figure 11, with Figure 11A showing the RUNX2 results, Figure 11B showing the CAP results, Figure 11C showing the BMP2 results and Figure 11D showing the OCN results.

The qPCR data showed that both LipSi and pSi particles activated osteogenic and cementogenic genes across all timepoints, at similar levels to what was observed using osteogenic medium.

### Example 16 - Bioactivity in vivo: regenerative capacity

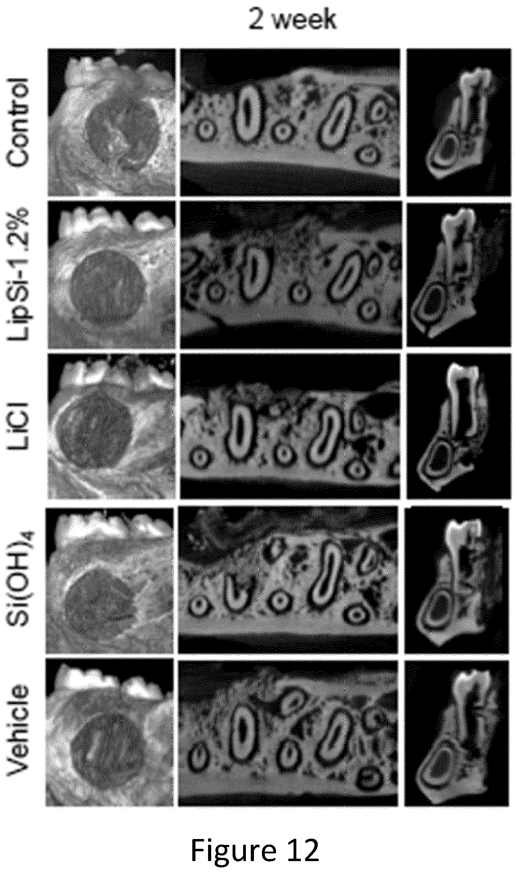

A mouse model of periodontal disease was used in this experiment. A 3mm diameter, 1.5mm depth cylindrical hole was made in the alveolar bone of the animal also inducing the loss of periodontal ligament and tooth cementum. The hole was filled with a Pluronic F127 gel carrier alone or incorporating either LipSi (SS21), porous silicon particles or lithium chloride. The defect was sealed using the BioGide^{®} guided tissue regeneration membrane. No treatment was used as control. At 2 weeks and 6 weeks the animals were sacrificed and the mandibles of the animals analysed my microCT to measure bone mineral density (BMD) and bone volume over total volume (BV/TV). Figure 12 shows exemplary microCT images at 2 weeks.

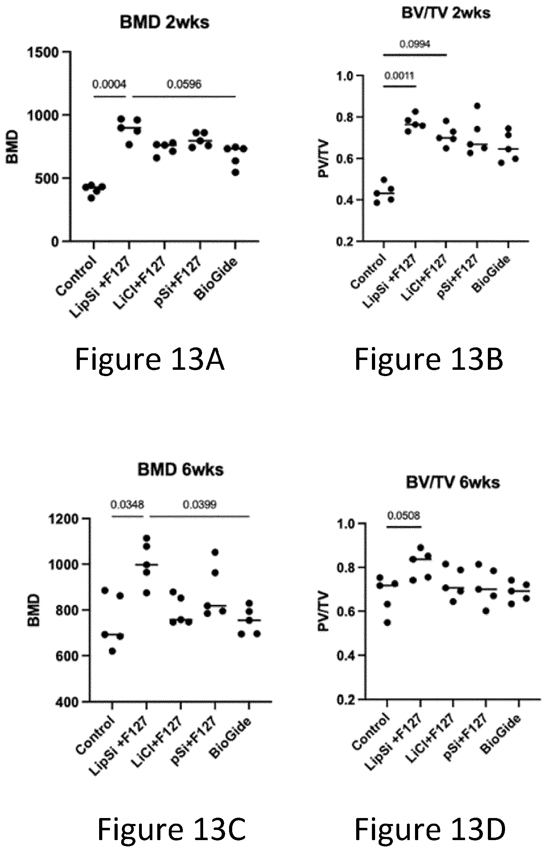

The LipSi sample showed the most bone regeneration of all the groups considered by both the BMD (Figure 13A - 2 weeks; Figure 13C - 6 weeks) and BV/TV (Figure 13B - 2 weeks; Figure 13D - 6 weeks) metrics at 2 and 6 weeks, and was the only group with a statistically significant difference with respect to the control. This data indicates that LipSi promote the regeneration of alveolar bone.

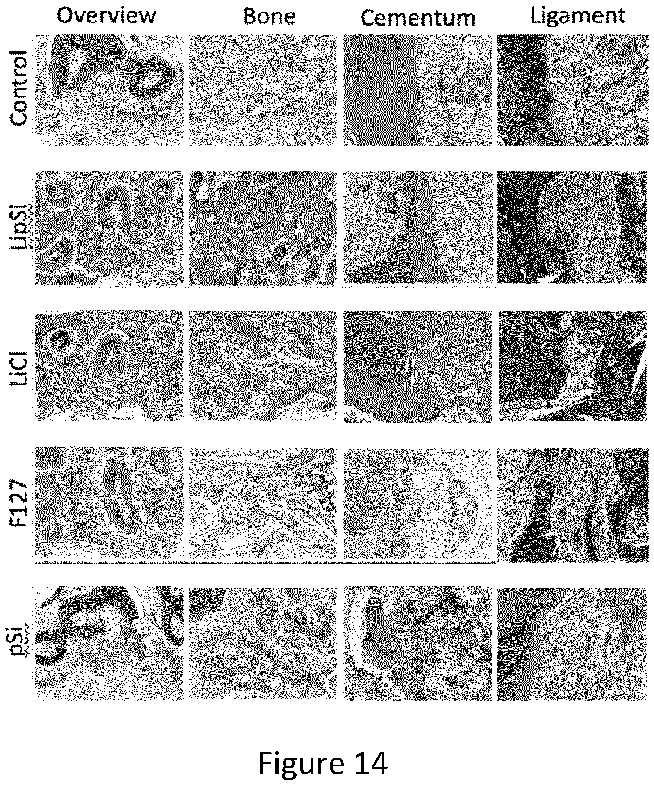

The mandibular samples from 2-weeks and 6-weeks were de-calcified and histological sections were prepared for H&E and masson staining and analysis. The results from the 2-week group are shown in Figure 14.

The histological analysis confirmed the improved regenerative potential of LipSi for bone, and also showed the regeneration of tooth cementum, highlighted by the formation of tertiary cementum, and of the fibrous structures of the periodontal ligament, highlighted by the new growth of cellularised tissue in the interstitial region between new bone and new cementum.

## Claims

1. Lithiated porous silicon particles comprising lithium, silicon and oxygen.

2. Lithiated porous silicon particles according to claim 1, wherein the particles are mesoporous.

3. Lithiated porous silicon particles according to claim 1 or claim 2, wherein the particles are bioresorbable.

4. Lithiated porous silicon particles according to any preceding claim, wherein each particle is monolithic.

5. A composition comprising the lithiated porous silicon particles according to any preceding claim, wherein the composition is a bone cement or a dental cement.

6. Lithiated porous silicon particles for use in therapy, wherein the particles comprise lithium and silicon.

7. Lithiated porous silicon particles for use according to claim 6, which are for use in the repair or regeneration of mineralised tissue or soft tissue, preferably mineralised tissue, more preferably mineralised bone or dental tissue.

8. Lithiated porous silicon particles for use according to claim 6 or claim 7, which are for use in the prevention or treatment of dental cavities, dental fissure, bone fractures, caries, periodontitis, periodontal ligament disorders, alveolar bone loss, craniofacial defects (e.g. cleft palate/alveolar ridge), bone loss, dentine loss, spinal cord injury and surgical bone removal.

9. A method of preparing lithiated porous silicon particles, the method comprising contacting porous silicon particles with a source of lithium in the presence of oxygen.

10. The method according to claim 9, wherein the method is carried out in a gaseous environment comprising at least 5 parts per million (ppm) of oxygen gas, or in an aqueous environment comprising at least 5 ppm of dissolved oxygen.

11. The method according to claim 9 or claim 10, wherein the method is carried out in the presence of at least 5 parts per million (ppm) of water.

12. The method according to any one of claims 9 to 11, wherein the porous silicon particles and the source of lithium are heated above room temperature, preferably to a temperature of from 50 °C to 1000 °C.

13. The method according to any one of claims 9 to 12, wherein the molar ratio of silicon to lithium used in the method is from 10:1 to 1:10.

14. The method according to any one of claims 9 to 13, wherein the source of lithium is selected from metallic lithium, lithium halides (e.g. lithium bromide, lithium chloride, lithium iodide, lithium fluoride), lithium hydroxide (LiOH), lithium carbonate (Li₂CO₃), lithium oxide (LiO₂), lithium peroxide (Li₂O₂), lithium nitrate, lithium sulphate, lithium phosphate, lithium citrate (Li₃C₆H₅O₇) and mixtures thereof.

15. The method according to any one of claims 9 to 14, wherein the porous silicon particles are mesoporous.

## Patentansprüche

1. Lithiumhaltige poröse Siliziumpartikel, die Lithium, Silizium und Sauerstoff umfassen.

2. Lithiumhaltige poröse Siliziumpartikel nach Anspruch 1, wobei die Partikel mesoporös sind.

3. Lithiumhaltige poröse Siliziumpartikel nach Anspruch 1 oder Anspruch 2, wobei die Partikel bioresorbierbar sind.

4. Lithiumhaltige poröse Siliziumpartikel nach einem vorhergehenden Anspruch, wobei jedes Partikel monolithisch ist.

5. Zusammensetzung, umfassend die lithiumhaltigen porösen Siliziumpartikel nach einem vorhergehenden Anspruch, wobei die Zusammensetzung ein Knochenzement oder ein Dentalzement ist.

6. Lithiumhaltige poröse Siliziumpartikel zur Verwendung bei Therapie, wobei die Partikel Lithium und Silizium umfassen.

7. Lithiumhaltige poröse Siliziumpartikel zur Verwendung nach Anspruch 6, die zur Verwendung bei der Reparatur oder Regeneration von mineralisiertem Gewebe oder Weichgewebe, bevorzugt mineralisiertem Gewebe, bevorzugter mineralisiertem Knochen oder Dentalgewebe sind.

8. Lithiumhaltige poröse Siliziumpartikel zur Verwendung nach Anspruch 6 oder Anspruch 7, die zur Verwendung bei der Vorbeugung oder Behandlung von Dentalkavitäten, Dentalfissur, Knochenbrüchen, Karies, Parodontitis, Parodontalbandstörungen, alveolärem Knochenverlust, kraniofazialen Defekten (z. B. Gaumenspalte/Alveolarkamm), Knochenverlust, Dentinverlust, Rückenmarksverletzung und chirurgischer Knochenentfernung sind.

9. Verfahren zum Herstellen von lithiumhaltigen porösen Siliziumpartikeln, wobei das Verfahren Kontaktieren von porösen Siliziumpartikeln mit einer Lithiumquelle in der Gegenwart von Sauerstoff umfasst.

10. Verfahren nach Anspruch 9, wobei das Verfahren in einer gasförmigen Umgebung, die zumindest 5 Teile pro Million (ppm) Sauerstoffgas umfasst, oder in einer wässrigen Umgebung, die zumindest 5 ppm gelösten Sauerstoff umfasst, durchgeführt wird.

11. Verfahren nach Anspruch 9 oder Anspruch 10, wobei das Verfahren in der Gegenwart von zumindest 5 Teilen pro Million (ppm) Wasser durchgeführt wird.

12. Verfahren nach einem der Ansprüche 9 bis 11, wobei die porösen Siliziumpartikel und die Lithiumquelle über Raumtemperatur, bevorzugt auf eine Temperatur von 50 °C bis 1000 °C, erhitzt werden.

13. Verfahren nach einem der Ansprüche 9 bis 12, wobei das Molverhältnis von Silizium zu Lithium, das in dem Verfahren verwendet wird, von 10:1 bis 1:10 ist.

14. Verfahren nach einem der Ansprüche 9 bis 13, wobei die Lithiumquelle ausgewählt ist aus metallischem Lithium, Lithiumhalogeniden (z. B. Lithiumbromid, Lithiumchlorid, Lithiumiodid, Lithiumfluorid), Lithiumhydroxid (LiOH), Lithiumcarbonat (Li₂CO₃), Lithiumoxid (LiO₂), Lithiumperoxid (Li₂O₂), Lithiumnitrat, Lithiumsulfat, Lithiumphosphat, Lithiumcitrat (Li₃C₆H₅O₇) und Mischungen davon.

15. Verfahren nach einem der Ansprüche 9 bis 14, wobei die porösen Siliziumpartikel mesoporös sind.

## Revendications

1. Particules de silicium poreux lithié comprenant du lithium, du silicium et de l'oxygène.

2. Particules de silicium poreux lithié selon la revendication 1, lesdites particules étant mésoporeuses.

3. Particules de silicium poreux lithié selon la revendication 1 ou la revendication 2, lesdites particules étant biorésorbables.

4. Particules de silicium poreux lithié selon une quelconque revendication précédente, dans lesquelles chaque particule est monolithique.

5. Composition comprenant les particules de silicium poreux lithié selon une quelconque revendication précédente, ladite composition étant un ciment osseux ou un ciment dentaire.

6. Particules de silicium poreux lithié destinées à être utilisées en thérapie, lesdites particules comprenant du lithium et du silicium.

7. Particules de silicium poreux lithié destinées à être utilisées selon la revendication 6, qui sont destinées à être utilisées dans la réparation ou la régénération de tissus minéralisés ou de tissus mous, de préférence de tissus minéralisés, idéalement d'os minéralisés ou de tissus dentaires.

8. Particules de silicium poreux lithié destinées à être utilisées selon la revendication 6 ou la revendication 7, qui sont destinées à être utilisées dans la prévention ou le traitement de cavités dentaires, de fissures dentaires, de fractures osseuses, de caries, de parodontites, de troubles du ligament parodontal, de la perte osseuse alvéolaire, d'anomalies crânio-faciales (par exemple fente palatine/crête alvéolaire), de perte osseuse, de perte de dentine, de lésion de la moelle épinière et d'ablation osseuse chirurgicale.

9. Procédé de préparation de particules de silicium poreux lithié, le procédé comprenant la mise en contact de particules de silicium poreux avec une source de lithium en présence d'oxygène.

10. Procédé selon la revendication 9, ledit procédé étant réalisé dans un environnement gazeux comprenant au moins 5 parties par million (ppm) d'oxygène gazeux, ou dans un environnement aqueux comprenant au moins 5 ppm d'oxygène dissous.

11. Procédé selon la revendication 9 ou la revendication 10, ledit procédé étant réalisé en présence d'au moins 5 parties par million (ppm) d'eau.

12. Procédé selon l'une quelconque des revendications 9 à 11, dans lequel les particules de silicium poreux et la source de lithium sont chauffées au-dessus de la température ambiante, de préférence à une température de 50 °C à 1000 °C.

13. Procédé selon l'une quelconque des revendications 9 à 12, dans lequel le rapport molaire du silicium au lithium utilisé dans le procédé est de 10:1 à 1:10.

14. Procédé selon l'une quelconque des revendications 9 à 13, dans lequel la source de lithium est choisie parmi le lithium métallique, les halogénures de lithium (par exemple, le bromure de lithium, le chlorure de lithium, l'iodure de lithium, le fluorure de lithium), l'hydroxyde de lithium (LiOH), le carbonate de lithium (Li₂CO₃), l'oxyde de lithium (LiO₂), le peroxyde de lithium (Li₂O₂), le nitrate de lithium, le sulfate de lithium, le phosphate de lithium, le citrate de lithium (Li₃C₆H₅O₇) et des mélanges de ceux-ci.

15. Procédé selon l'une quelconque des revendications 9 à 14, dans lequel les particules de silicium poreux sont mésoporeuses.
